# EUROPEAN PATENT APPLICATION

(11) **EP 2 487 176 A1**
(43) Date of publication of application: **15.08.2012**
(21) Application number: 11154265.0
(22) Date of filing: 14.02.2011
(51) Int. Cl.: C07D 471/18, C07D 513/18, C07D 471/22, C07D 498/22, C07D 515/18, A61K 31/439, A61K 31/4995, A61P 31/14

(54) **Macrocyclic integrase inhibitors for use in the treatment of feline immunodeficiency virus**

(71) Applicant: Elanco Animal Health Ireland Limited, Dublin (IE)
(72) Inventor: Thuring, Johannes Wilhelmus John F., 2018 Antwerpen (BE)
(74) Representative: Bassinder, Emma Marie

(57) **Abstract**

This invention concerns to naphthyridin derivatives of formula (I) for use in the treatment or prevention of feline immunodeficiency virus (FIV). Furthermore the present invention relates to a method of treating or preventing infection of feline immunodeficiency virus in a feline animal wherein said method comprises the administration to said feline animal of a therapeutically effective amount of a naphthyridin derivative.

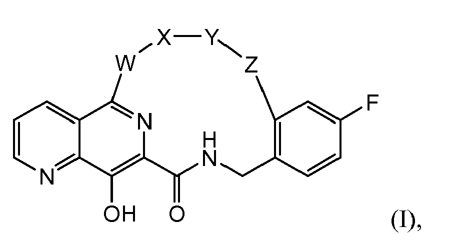

## Description

This invention concerns to naphthyridin derivatives for use in the treatment or prevention of feline immunodeficiency virus (FIV). Furthermore the present invention relates to a method of treating or preventing infection of feline immunodeficiency virus in a feline animal wherein said method comprises the administration to said feline animal of a therapeutically effective amount of a naphthyridin derivative.

Feline immunodeficiency virus (FIV) is a lentivirus that affects domesticated housecats worldwide and is the causative agent of feline AIDS. The primary modes of FIV transmission are deep bite wounds and scratches, where the infected cat's saliva enters the other cat's bloodstream. FIV may also be transmitted from pregnant females to their offspring *in utero.* FIV can attack the immune system of cats, much like the human immunodeficiency virus (HIV) can attack the immune system of human beings. Cats can tolerate FIV infection reasonably well but eventually FIV infection can lead to debilitation of the immune system whereby the infected cat eventually succumbs to chronic infections due to a suppressed immune system function.

The present invention is aimed at providing a particular group of naphthyridine derivatives for use in the treatment or prevention of feline immunodeficiency virus in a feline animal.

Thus, in one aspect, the present invention concerns compounds of formula (I), including the stereochemically isomeric forms thereof, and pharmaceutically acceptable salts thereof, which can be represented by formula (I) : 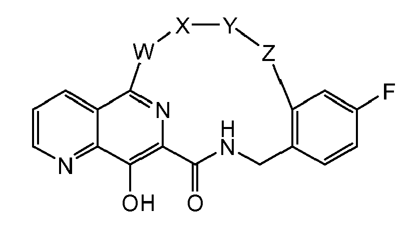
wherein W is -NH-, -N(CH₃)- or piperazine,
X is a bond, -C(=O)- or ―S(=O)₂-,
Y is C₃₋₇alkylene,
Z is -NH-C(=O)- or -O-;
for use in the treatment or prevention of feline immunodeficiency virus.

Whenever used in a molecular fragment or group, a dashed line - - - represents the bond linking that fragment or group with the remainder of the molecule.

As used herein C₃-₇alkylene as a group or part of a group defines straight or branched bivalent chain saturated hydrocarbon radicals having from 3 to 7 carbon atoms such as propylene, 2-propyl, 1-butylene, propylene, hexylene or heptylene. Of interest among C₃₋₇alkylene is C₄₋₅alkylene or C₃₋₄alkylene; C₄₋₅alkylene defines straight or branched bivalent chain saturated hydrocarbon radicals having from 4 or 5 carbon atoms; C₃₋₄alkylene defines straight or branched bivalent chain saturated hydrocarbon radicals having from 3 or 4 carbon atoms. Of interest are those alkylene radicals being straight.

Whenever a radical occurs in the definition of the compounds of formula (I) or in any of the subgroups specified herein, said radical independently is as specified above in the definition of the compounds of formulas (I) or in the more restricted definitions as specified hereinafter.

It should also be noted that the radical positions on any molecular moiety used in the definitions may be anywhere on such moiety as long as it is chemically stable. For instance butyl includes 1-butyl and 2-butyl.

Some of the compounds of formula (I) may also exist in their tautomeric form. Such forms although not explicitly indicated in the above formula are intended to be included within the scope of the present invention.

The present invention is also intended to include any isotopes of atoms present in the compounds of the invention. For example, isotopes of hydrogen include tritium and deuterium and isotopes of carbon include C-13 and C-14.

Whenever used hereinabove or hereinafter, the terms "compounds of formula (I)", "the present compounds", "the compounds of the present invention" or any equivalent terms, and similarly, the terms "subgroups of compounds of formula (I)", "subgroups of the present compounds", "subgroups of the compounds of the present invention" or any equivalent terms, are meant to include the compounds of general formula (I), or subgroups of the compounds of general formula (I), as well as their salts, solvates, and stereoisomers.

Subgroups of compounds of formula I are those compounds of formula I, or subgroups of compounds of formula I, as defined herein,
- wherein W is -NH- or -N(CH₃)-, or,
- wherein X is a bond or -S(=O)₂-, or,
- wherein Y is C₄₋₅alkylene, or,
- wherein X is -C(=O)- and Y is C₃₋₄alkyl when W is piperazine, or,
- wherein Z is -O-, or,
- wherein Z is -NH-C(=O)-, in particular wherein the nitrogen of -NH-C(=O)- is connected to Y, or
- wherein the -W-X-Y-Z- linker is 8 or 9 atoms long, or,
- wherein -W-X-Y-Z- is selected from:

   ---NH-C₅₋₇akylene-NH-C(=O)---,

   ---N(CH₃)-S(=O)₂-C₄₋₅alkylene-NH-C(=O)---,

   ---N(CH₃)-S(=O)₂-C₄₋₅alkylene-O---,

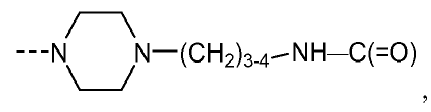

   and,

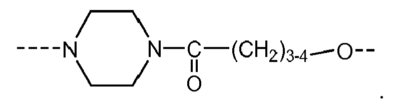

The pharmaceutically acceptable addition salt forms, which the compounds of the present invention are able to form, can conveniently be prepared using the appropriate acids, such as, for example, inorganic acids such as hydrohalic acids, e.g. hydrochloric or hydrobromic acid, sulfuric, hemisulphuric, nitric, phosphoric, and the like acids; or organic acids such as, for example, methanesulfonic, ethanesulfonic, benzenesulfonic, p-toluenesulfonic, and the like acids. Conversely said acid addition salt forms can be converted into the free base form by treatment with an appropriate base.

The compounds of formula (I) containing acidic protons may be converted into their pharmaceutically acceptable metal or amine addition salt forms by treatment with appropriate organic and inorganic bases. Appropriate base salt forms comprise, for example, the ammonium salts, the alkali and earth alkaline metal salts, e.g. the lithium, sodium, potassium, magnesium, calcium salts and the like, salts with organic bases, e.g. primary, secondary, and tertiary aliphatic and aromatic amines such as methylamine, ethylamine, propylamine, isopropylamine, the four butylamine isomers, dimethyl-amine, diethylamine, diethanolamine, dipropylamine, diisopropylamine, di-n-butylamine, pyrrolidine, piperidine, morpholine, trimethylamine, triethylamine, tripropylamine, quinuclidine, pyridine, quinoline and isoquinoline, the benzathine, *N*-methyl-D-glucamine, 2-amino-2-(hydroxymethyl)-1,3-propanediol, hydrabamine salts, and salts with amino acids such as, for example, arginine, lysine and the like. Conversely the salt form can be converted by treatment with acid into the free acid form.

### Preparation of the compounds

A compound according to the invention can generally be prepared by a succession of steps, each of which is known to the skilled person. In particular, the compounds in this patent application can be prepared according to one or more of the following preparation methods. In the following schemes, and unless otherwise indicated, all variables used are as defined for compounds of formula (I).

The macrocycles with the general formula (I) of the present invention can be prepared through a cyclization reaction involving an "open" precursor of the general formula (II), in which the 8-hydroxyl function of the 1,6-naphthyridine is protected with a protecting group (PG), or, alternatively, is kept unprotected and used as the free hydroxyl function. Examples of suitable protecting groups for said hydroxyl function are, C₁₋₄alkyl, benzyl, aryl sulfonyl, and benzoyl. Said cyclization can be effected through the formation an amide bond, involving the carboxylic acid function at the 7-position of the naphthyridine scaffold, as is shown in Scheme 1, and requires the presence of a dehydrating reagent. Commonly used examples are HBTU (O-benzotriazole-*N,N,N',N'* -tetramethyl uronium hexafluorophosphate), EDCI (1-ethyl-3-(3-dimethylaminopropyl)carbodiimide)(1-ethyl-3-(3-dimethylaminopropyl)carbodiimide), EDAC (1-ethyl-3-(3-dimethylaminopropyl) carbodiimide hydrochloride), or FDPP (pentafluorophenyl diphenylphosphinate). In a particular embodiment said dehydrating reagent is HBTU or FDPP. The reaction is typically performed by slow addition of the open precursor of the general formula (II) to a mixture containing said dehydrating agent and an excess amount of a tertiary amine, such as diisopropyl ethyl amine, or the like. A useful solvent is an aprotic solvent like CH₂Cl₂, or more preferably a polar aprotic solvent like DMF. Under certain circumstances the use of HOBT (hydroxybenzotriazole) as an additive is an advantage. In a preferred embodiment the cyclization reaction is carried out at low concentration of the open precursor (II), such as in the range between 1 and 10 mM, in particular at 4 mM.

In another embodiment, the macro cyclization reaction can be effected in the linker region -W-X-Y-Z-, for example in the preparation of compounds of formula I wherein Z represents an amide bond of formula -NH-C(=O)- wherein intermediate of formula IIa is cyclized using a methodology as described hereinbefore to form a macrocyclic amide bond.

The deprotection of the 8-hydroxyl group of compounds of formula III as illustrated in Scheme 1 can be affected using various conditions, and depends on the particular protecting group PG. In one embodiment, when PG is benzyl, a macrocycle of the general formula (III) can be treated with trifluoro acetic acid at a temperature between 0°C and 80°C. Optionally, an aprotic co-solvent such as dichloro methane can be advantageously used. It might also be advantageous to apply an agent that traps the resulting benzylic carbocation, for example triisopropyl silane, or the like. In a second embodiment, when PG is Me, the macrocycle of the general formula (III) can be treated with sodium iodide and tetrachloro silane in a solvent mixture consisting of a polar aprotic solvent, such as acetonitrile or the like, and an aromatic apolar solvent, such as toluene or the like. Said transformation is advantageously carried out in a temperature range between 0°C and room temperature. Alternatively, said deprotection is carried out using a boron reagent, such as boron tribromide (BBr₃), in an aprotic solvent such as dichloro methane, or the like, at low temperature, such as at -78°C. In a third embodiment, when PG is *para* tolyl sulfonyl, a macrocycle of the general formula (III) can be treated with a sodium alkoxide in the corresponding alcoholic solvent, eg sodium methoxide in methanol, at room temperature. Optionally a polar aprotic co-solvent can be applied, such as DMF, or the like.

The compounds of formula (II) are obtained from compounds of formula IV, and compounds of formula (Iia) from compounds of formula VI, by using appropriate deprotection methods as described for and illustrated by Scheme 2 and Scheme 2a respectively.

Various methods to obtain (subgroups) of intermediate compounds of formulas (IV) or (VI) starting from a compound of formula (XI) are described for and illustrated by schemes 3, 4, 4a, 4b, 4c, 5a, 5b and 5c.

The naphthyridine of the general formula (XI) can be prepared with different protecting groups (PG). The choice of PG depends on the particular functional groups A, B, C and D, as defined hereinbefore. The PG is installed on the hydroxyl napththyridine of the general formula (XIa), as is shown in Scheme 1. When PG is benzyl or C₁₋₄alkyl, said naphtyridine (XIa) is treated with an inorganic base, such as cesium carbonate or the like, in a polar, aprotic solvent, such as DMF, or the like, followed by addition of a C₁₋₄alkyl halide, such as methyl iodide, or benzyl bromide to afford the intermediate of the general formula (XI). The reaction is most advantageously carried out at room temperature. Alternatively, when PG = *para* tolyl sulfonyl, said naphtyridine (XIa) is treated with *para* toluene sulfonyl chloride, in the presence of a tertiary amine base, such as triethyl amine, or the like. A suitable solvent is a chlorinated hydrocarbon, such as chloroform, or the like, and the reaction temperature should be between 20°C and 50°C.

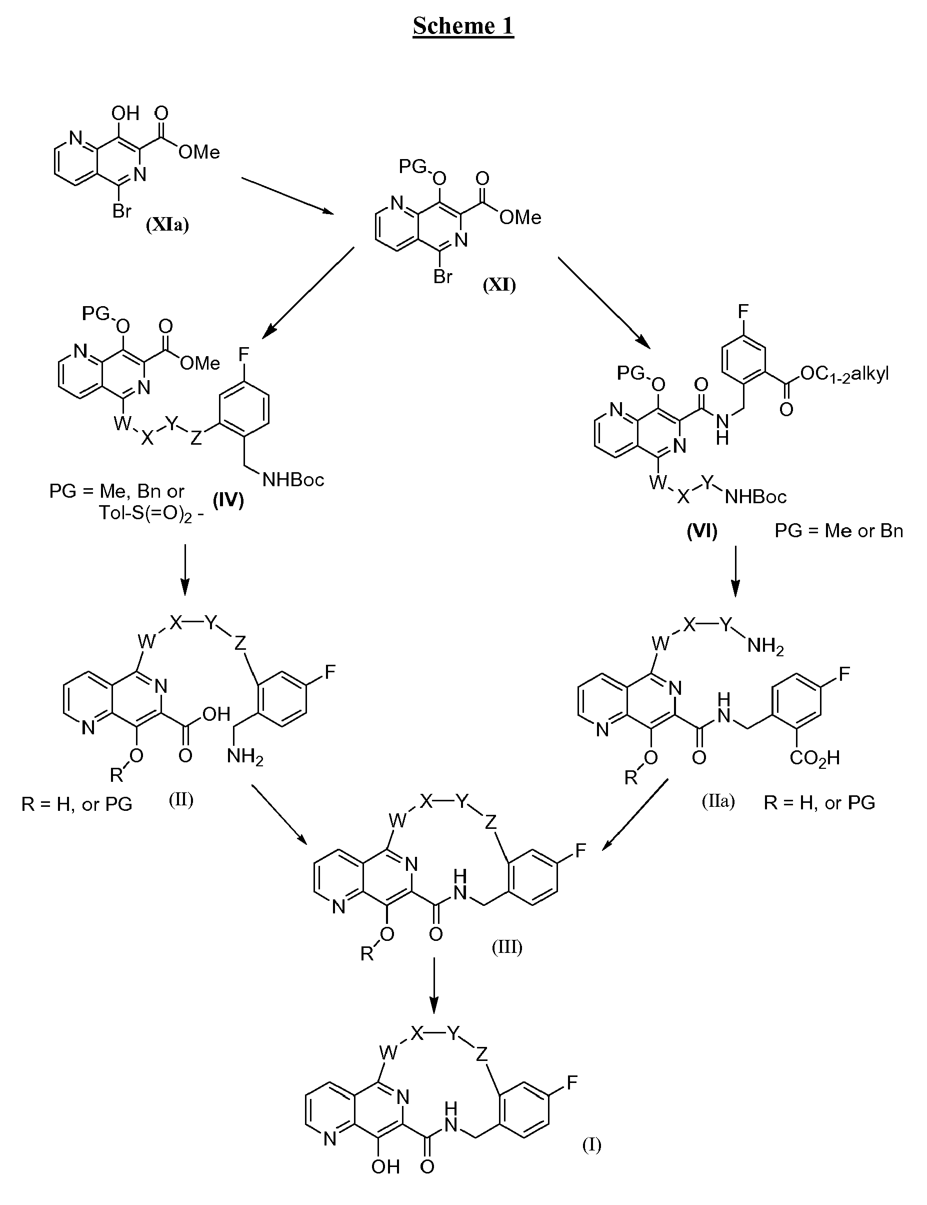

The open precursors of the general formula (II) can be prepared in two steps starting from the protected precursor of the general formula (IV), as is shown in Scheme 2: First, the carboxylic ester of the general formula (IV) is saponified to yield the corresponding carboxylic acid of the general formula (V). This transformation can be effected by using a metal hydroxide (M-OH), such as potassium hydroxide, sodium hydroxide, or lithium hydroxide. The reaction is performed in an aqueous environment, and is most advantageously carried out in the presence of at least one water miscible organic co-solvent, such as methanol, ethanol or THF, or the like. In a second step, the amine Boc protecting group was removed to afford the macrocyle precursor of the general formula (II). This can be achieved by treating the Boc intermediate of formula (V) with a solution containing trifluoro acetic acid, optionally in the presence of triisopropyl silane, in an aprotic solvent, such as dichloro methane, or the like. In a preferred embodiment, said transformation is carried out between 0°C and room temperature. Alternatively, said deprotection can be effected by treatment of (V) with a solution of hydrochloric acid in a polar, aprotic solevent, such as dioxane, in particular with a 4N solution ofHCl in dioxane.

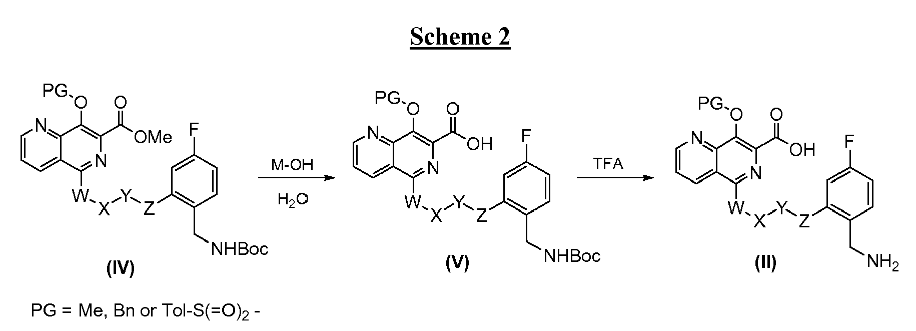

The open precursors of the general formula (IIa) can be prepared in two steps starting from the protected precursor of the general formula (VI), as is shown in Scheme 2a :

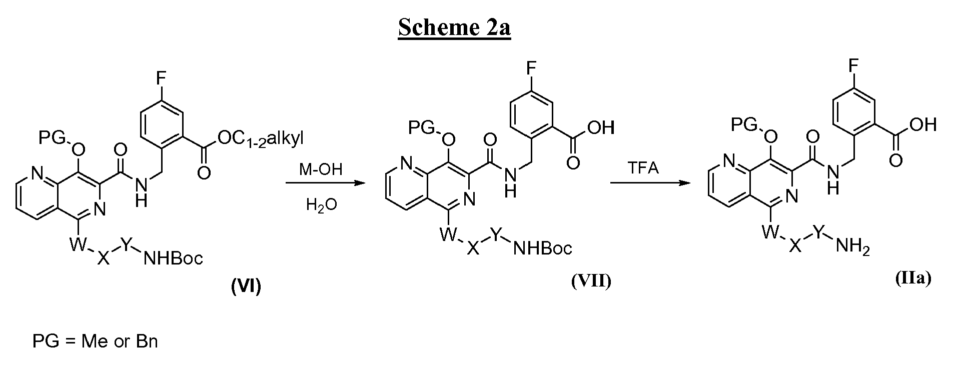

First, the carboxylic ester of the general formula (VI) is saponified to yield the corresponding carboxylic acid of the general formula (VII). This transformation can be effected by using a metal hydroxide (M-OH), such as potassium hydroxide, sodium hydroxide, or lithium hydroxide. The reaction is performed in an aqueous environment, and is most advantageously carried out in the presence of at least one water miscible organic co-solvent, such as methanol, ethanol or THF, or the like. In a second step, the amine Boc protecting group was removed to afford the macrocyle precursor of the general formula (IIa). This can be achieved by treating the Boc intermediate of formula (VII) with a solution containing trifluoro acetic acid, optionally in the presence of triisopropyl silane, in an aprotic solvent, such as dichloro methane, or the like. In a preferred embodiment, said transformation is carried out between 0°C and room temperature.

The precursor of the general formula (VI) can be prepared in two steps from the naphthyridine of the general formula (VIII), as is depicted in Scheme 3. The first step involves the saponification of intermediate (VIII), which can be carried out by reaction with a metal hydroxide (M-OH), such as potassium hydroxide, or sodium hydroxide. The reaction is performed in an aqueous environment, and is most advantageously carried out in the presence of at least one water miscible organic co-solvent, such as methanol, or the like. Further conversion of the carboxylic acid (IX) into the amides of formula (VI) is done using art known procedures, such as the treatment with the hydrochloric acid salt of the primary amine of the formula (X), such as methyl 2-(aminomethyl)-5-fluorobenzoate hydrochloride when C₁₋₂alkyl is methyl, in the presence of a conventional amide coupling reagent such as HBTU (O-benzotriazole-*N,N,N',N'*-tetramethyl uronium hexafluorophosphate), EDCI (1-ethyl-3-(3-dimethylaminopropyl)carbodiimide), or EDAC (1-ethyl-3-(3-dimethylaminopropyl) carbodiimide hydrochloride) in an aprotic solvent like CH₂Cl₂, in the presence of an amine base additive, such as diisopropyl ethyl amine. Under certain circumstances the use of HOBT (hydroxybenzotriazole) as an additive is an advantage.

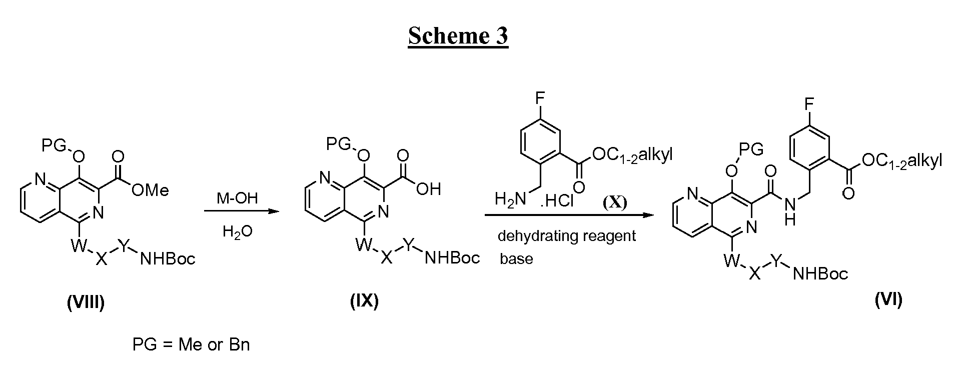

The intermediate of the general formula (VIIIf) can be prepared in several ways, depending on the nature of the functional groups W and X, as defined hereinbefore. In a first embodiment, when W is -NH-, and X is a bond, the reaction as shown in Scheme 4 can be used. Suitable protecting groups (PG) in this reaction are Me and Bn. Said transformation involves the use of a mono-protected bis-amine linker (XII). More particularly, said protecting group is a Boc group. Said linker can be introduced by treating a mixture of the bromide (XI) and a tertiary amine, such as diisopropyl ethyl amine, or the like, in a polar, aprotic solvent, such as DMA, with the amine (XII). The reaction is most advantageously carried out in a temperature range 80-160°C, in particular at 140°C, to afford the carboxylic ester of the general formula (VIIIf).

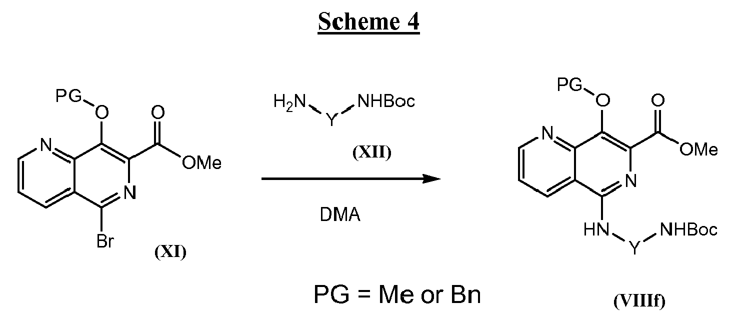

In a second embodiment, when W is piperazinyl, and X is a bond, the sequence as shown in Scheme 4a can be followed. In a first step, piperazine is incorporated in the naphthyridine of the general formula (XI). This can be done by treating a mixture of the bromide (XI) and a tertiary amine, such as diisopropyl ethyl amine, or the like, in a polar, aprotic solvent, such as DMA, with piperazine. The reaction is most advantageously carried out in a temperature range 80- 140°C, in particular at 110°C, to afford the piperazine of the general formula (XIII). In a second step, said piperazine (XIII) is treated with a bromo containing linker of the general formula (XIV). For example, when Y is C₄alkyl, said linker is *tert*-butyl 4-bromobutylcarbamate. This transformation requires the presence of an inorganic base, such as potassium carbonate, or the like. The reaction is advantageously carried out in a polar aprotic solvent, such as DMA, or the like, and affords the naphthyridine of the general formula (VIIIa).

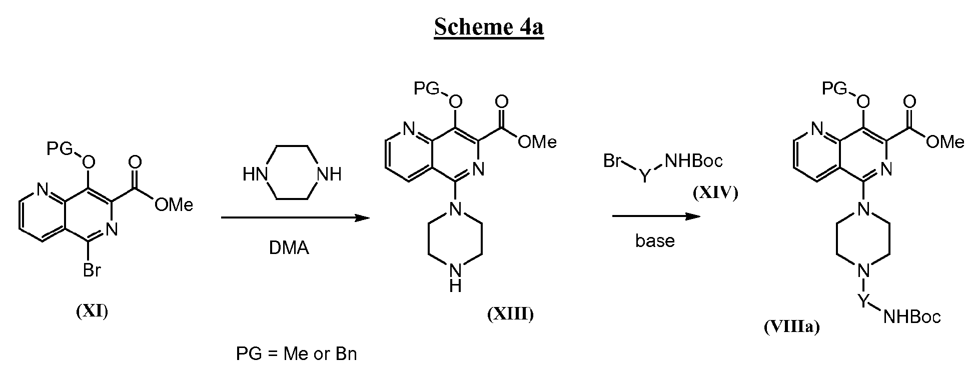

In a third embodiment, when W is -N(CH₃)-, X is -S(=O)₂-, Y is C₃₋₇alkylene, and Z is -NH-C(=O)-, the reaction as described in Scheme 4b can be followed, using the protected bromo naphthyridine of the general formula (XI). Suitable protecting groups are methyl or tosyl. The functionalized naphthyridine of the general formula (VIIIb) is prepared by reacting the bromo naphthyridine (XI) with the protected amino functionalized linker of the general formula (XV). The use of a copper(I) base, such as copper(I) oxide, in the presence of a ligand, such as 2,2'-bipyridine offer advantages in this reaction. Useful solvents are polar, aprotic solvents, such as DMA, NMP, or the like. The reaction needs elevated temperatures, typically in the range between 80°C and 140°C, in particular 120°C.

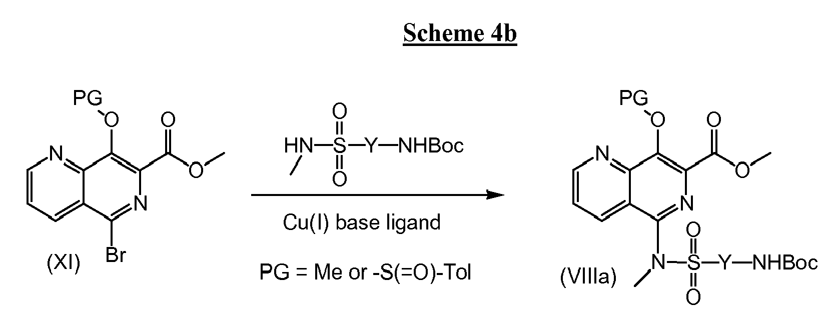

In certain cases it is advantageous to carry out a protecting group (PG) transformation of the 8-hydroxy function of the naphthyridine. An example of such is a strategy is the interconversion of the tosyl group in (VIIIc) into a benzyl group in (VIIIe), as is shown in Scheme 4c. The deprotection step involves treatment of the tosylate (VIIIc) with a sodium alkoxide in the corresponding alcoholic solvent, eg sodium methoxide in methanol. The reaction temperature is between 20 and 60°C. Optionally a polar aprotic co-solvent can be applied, such as DMF, or the like. The re-protection to afford the benzyl oxy napththyridine of the general formula (VIIIe) can be performed by treating (VIIId) with an inorganic base, such as cesium carbonate or the like, in a polar, aprotic solvent, such as DMF, or the like, followed by addition of a benzyl halide, such as benzyl bromide. The reaction is most advantageously carried out at room temperature.

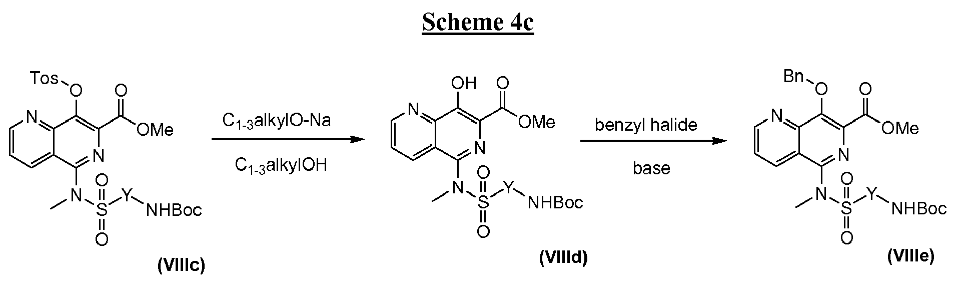

The macrocycle precursors of the general formula (IV) can be prepared in several ways, depending on the nature of the functional groups W, X and Z, as defined hereinbefore.

In a first embodiment, when W is piperazinyl, X is -C(=O)-, and Z is an oxygen atom the reaction as shown in Scheme 5a can be followed to afford the macrocycle precursor of the general formula (IVa): The piperazine of the general formula (XIII) is treated with the carboxylic acid of the general formula (XVI), in the presence of a conventional amide coupling reagent such as HBTU (O-benzotriazole-*N,N,N',N*'-tetramethyl uronium hexafluorophosphate ), EDCI (1-ethyl-3-(3-dimethylaminopropyl)-carbodiimide), or EDAC (1-ethyl-3-(3-dimethylaminopropyl) carbodiimide hydrochloride) in an aprotic solvent like CH₂Cl₂, in the presence of an amine base additive, such as diisopropyl ethyl amine.

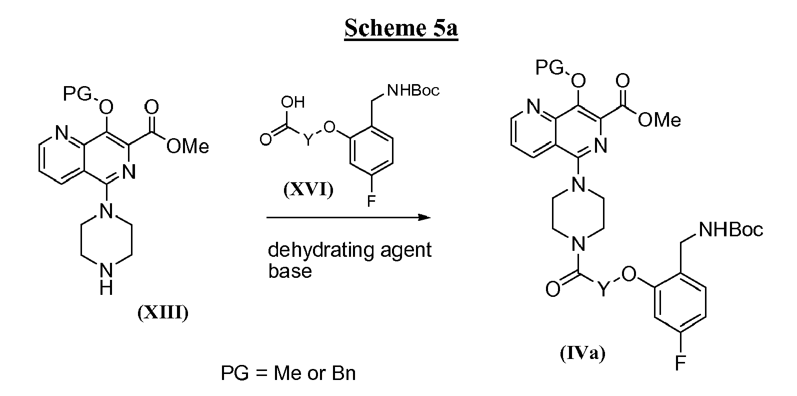

In a second embodiment, when W is -N(CH₃)-, X is -S(=O)₂-, Y is C₃₋₇alkylene, and Z is -NH-C(=O)-, the sequence as described in Scheme 5b can be followed, starting from the Boc-amino functionalized naphthyridine of the general formula (VIIIb), to afford the macrocyle precursor of the general formula (IVb). First, deprotection of the Boc-amino group in (VIIIb) can be achieved by treatment with TFA to afford the amine of the formula (XVIII). Optionally, a halogenated hydrocarbon can be used as a cosolvent, and the reaction temperature is between 0 and 20°C. Alternatively, said deprotection can be effected by using HCl in a polar, aprotic solvent, such as dioxane. In a second step, the amine of the general formula (XVIII) is treated with the carboxylic acid (XVII) in the presence of a conventional amide coupling reagent such as HBTU (O-benzotriazole-*N,N,N',N*' -tetramethyl uronium hexafluorophosphate), EDCI (1-ethyl-3-(3-dimethylaminopropyl)carbodiimide), or EDAC (1-ethyl-3-(3-dimethylaminopropyl) carbodiimide hydrochloride) in an aprotic solvent like CH₂Cl₂, or alternatively in a polar, aprotic solvent, such as DMF, or the like, in the presence of an amine base additive, such as diisopropyl ethyl amine.

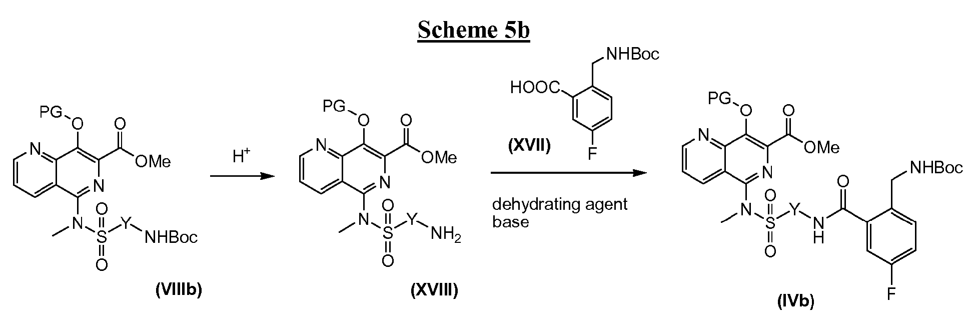

In a third embodiment, when W is -(NCH₃)-, X is -S(=O)₂-, Y is C₃₋₇alkylene, and Z is an oxygen atom, the reaction as depicted in Scheme 5c can be used, starting from the bromo naphthyridine of the general formula (XI), to afford the macrocyle precursor of the general formula (IVc). Suitable protecting groups are methyl or tosyl. The macrocycle precursor of the general formula (IVc) is prepared by reacting the bromo naphthyridine (XI) with the sulfonamide containing linker of the general formula (XIX). The use of a copper(I) base, such as copper(I) oxide, in the presence of a ligand, such as 2,2'-bipyridine offer advantages in this reaction. Useful solvents are polar, aprotic solvents, such as DMA, NMP, or the like. The reaction needs elevated temperatures, typically in the range between 80°C and 140°C, in particular 120°C, and is generally performed in an inert atmosphere.

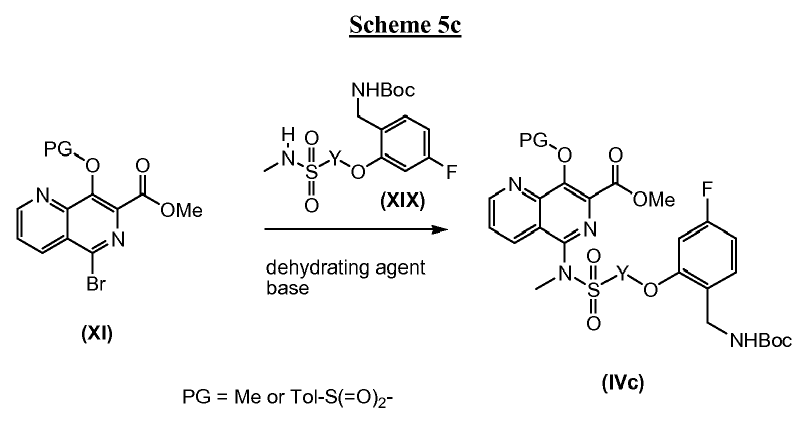

To assemble the advanced intermediates and macrocyles as described in Schemes 1-5c, the building blocks as depicted in Scheme 7a - 9, can be advantageously used. Said building blocks can be prepared according to the descriptions as provided hereinafter:
The Boc-amino functionalized linker of the general formula (XV) can be prepared from the amino sulfonamide of the general formula (XV'), as is depicted in Scheme 7a.
   Typically, a solution of the amine (XV') in a chlorinated hydrocarbon solvent, such as dichloromethane, or the like, is treated with Boc anhydride, at room temperature.

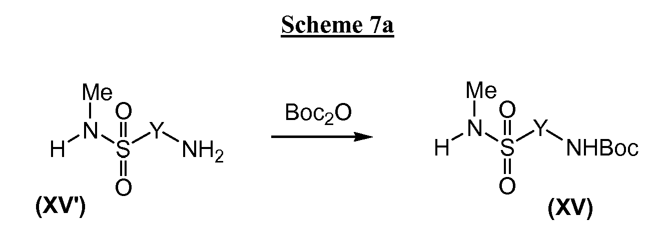

The amino functionalized linker of the general formula (XV') can be prepared from the chloro sulfonamide (XVa') in different ways, and depends on the nature of Y, as defined hereinbefore. For example, when Y is C₃₋₄alkyl, leading to the linker of the formula (XVb), the sequence as depicted in Scheme 7b can be followed. The chloro sulfonamide (XVa') is first treated with sodium iodide, in a polar, aprotic solvent, such as DMF, or the like, at room temperature. Next, sodium azide is added and the mixture is allowed to react in a temperature range between 20 and 70°C, in particular at 60°C, to afford the azide (XVc). In a next step, the azide is reduced to afford the amine (XVd). This transformation can be effected by putting the azide (XVc) under a hydrogen atmosphere, typically at 1 atm., in a protic solvent, such as methanol, or the like. The use of a catalyst, such as palladium on carbon, or the like, is essential to affect said hydrogenation reaction.

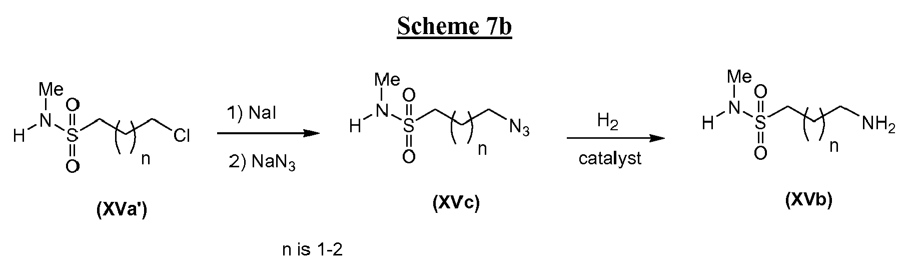

In another example, when Y is pentylene, leading to the linker of the formula (XVd), the sequence as depicted in Scheme 7c can be followed. The chloro sulfonamide (XVa) is first treated with sodium iodide, in a polar, aprotic solvent, such as DMF, or the like, at room temperature. Next, sodium cyanide is added and the mixture is allowed to react in a temperature range between 20 and 70°C, in particular at 60°C, to afford the nitrile (XVe). In a next step, the nitrile is reduced to afford the amine (XVd). This transformation can be effected by putting the nitrile (XVe) under a hydrogen atmosphere, typically at 1 atm., in a protic solvent, such as methanol, or the like, in the presence of ammonia. The use of a catalyst, such as palladium on carbon, or the like, is essential to effect said hydrogenation reaction. Alternatively, the reduction of the nitrile function in (XVe) can be effected by treatment with borane dimethylsulfide complex at room temperature, in a polar aprotic solvent, such as THF, or the like, to afford the primary amine (XVd).

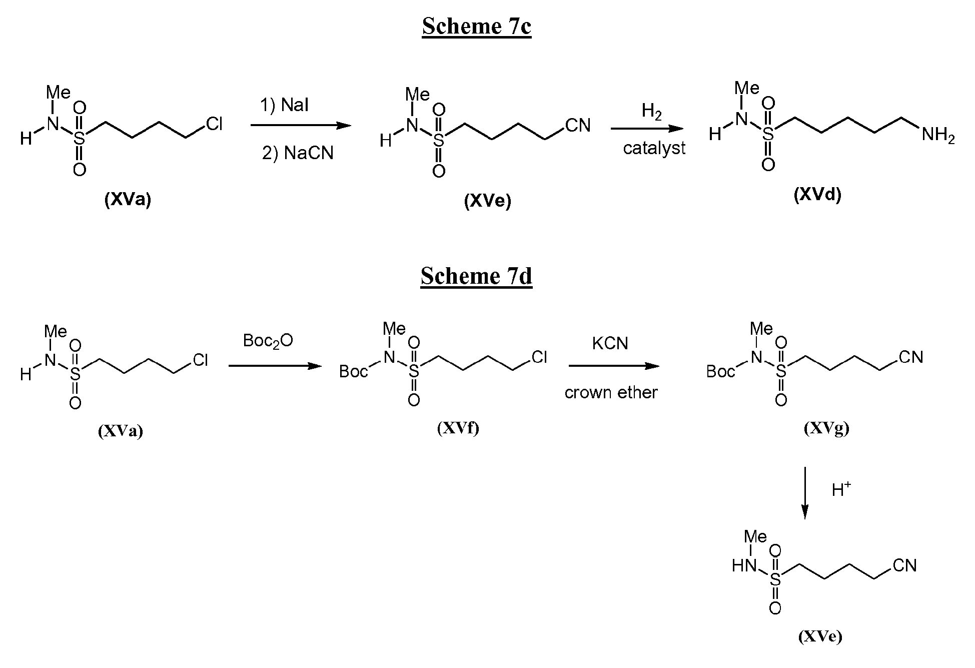

The nitrile of the formula (XVe) can also be prepared from the chloride (XVa) in three steps as is depicted in scheme 7d. The first step involves protection of the sulfonamide function with a suitable protecting group, such as Boc. This can be effected by treatment of the chloride (XVa) with Boc₂O at room temperature in a polar, aprotic solvent, such as DMF, or the like, to afford the Boc protected chloride (XVf). Optionally, a protic co-solvent can be used, such as methanol, or the like. The second step involves nucleophilic displacement of the chloride in (XVa) by cyanide. This can be effected by treatment of the chloride (XVa) with an inorganic cyanide salt, such as potassium cyanide, between room temperature and 150°C, in particular at 80°C, and, in a polar aprotic solvent, such as acetonitrile, or the like. It is advantageous to use a crown ether in this transformation, in particular 18-crown-6, to afford the nitrile of the formula (XVg). In a third step, deprotection of the Boc-amino group in (XVg) can be achieved by treatment with TFA to afford the cyanide of the formula (XVe). Optionally, a halogenated hydrocarbon can be used as a cosolvent, and the reaction temperature is between 0 and 20°C. Alternatively, said deprotection can be effected by using HCl in a polar, aprotic solvent, such as dioxane.

The carboxylic acid of the general formula (XVI) can be prepared in four steps from (2-(benzyloxy)-4-fluorophenyl)methanamine (XVIa), as is depicted in Scheme 8. In a first step the amine (XVIa) is protected with a Boc group. This can be effected by treatment of the amine (XVIa) in a solvent mixture consisting of dioxane and water, with Boc anhydride, in the presence of sodium carbonate, to obtain the Boc protected compound (XVIb). This reaction can be carried out in a temperature range between 0 and 20°C.

In a second step the benzyl group in (XVIb) is reductively removed by a reaction under a hydrogen atmosphere, typically at 1 atm., in a protic solvent, such as ethanol, or the like, optionally in the presence of an aprotic co-solvent, such as ethyl acetate, or the like. The use of a catalyst, such as palladium on carbon, or the like, is essential to effect said hydrogenation reaction, that affords the phenol (XVIc).

In a third step the phenol (XVIc) is reacted with a halo functionalized carboxylic ester of the general formula (XX), in the presence of an inorganic base, such as potassium carbonate, or the like. This transformation can be effected in a polar, aprotic solvent, such as DMA, or the like, in a temperature range between 20 and 80°C, in particular at 60°C, and affords the carboxylic ester of the general formula (XVId).

In a fourth step, the carboxylic ester of the general formula (XVId) is saponified to afford the carboxylic acid of the general formula (XVI). This transformation can be carried out by reaction with a metal hydroxide (M-OH), such as potassium hydroxide, or sodium hydroxide. The reaction is performed in an aqueous environment, and is most advantageously carried out in the presence of at least one water miscible organic co-solvent, such as methanol, or the like, and optionally THF.

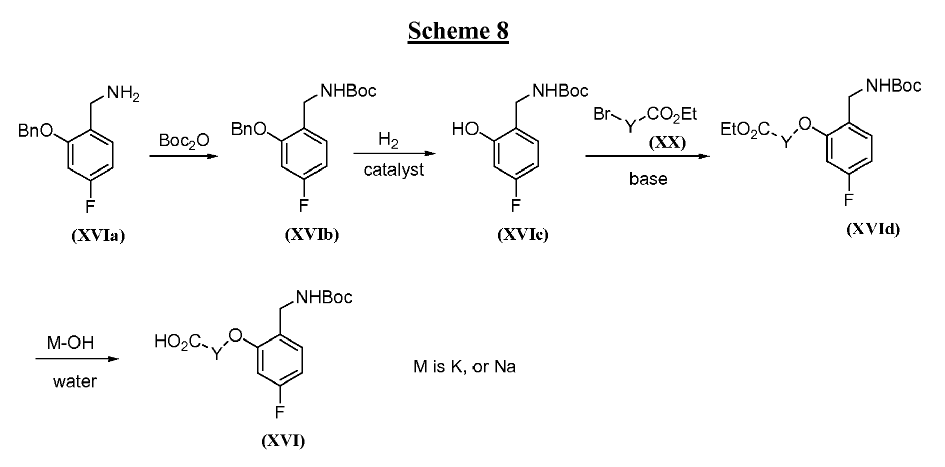

The sulfonamide building block of the general formula (XIX) can be prepared from the phenol (XVIc) and the sulfonamide of the general formula (XV") as is shown in Scheme 9a. The sulfonamide of the general formula (XV") contains a leaving group A, which can be halo, in particular iodo, or alternatively a tosylate. The phenol (XVIc) is reacted with (XV") in the presence of an inorganic base, such as potassium carbonate, or the like. This transformation can be effected in a polar, aprotic solvent, such as DMF, or DMSO, or the like, in a temperature range between 20 and 80°C, in particular at 50 - 60°C, and affords the sulfonamide of the general formula (XIX).

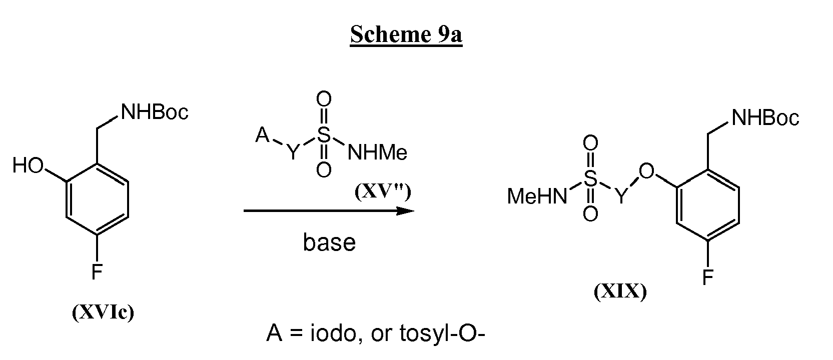

The sulfonamide linker of the general formula (XV") can be prepared using different methods, that depends on the nature of Y. For example, when Y is C₃₋₄alkyl, the iodo sulfonamide of the formula (XVa") can be prepared by reaction of the corresponding chloro sulfonamide (XVa'), with sodium iodide, as is shown in Scheme 9b. This transformation is performed in acetone at reflux temperature.

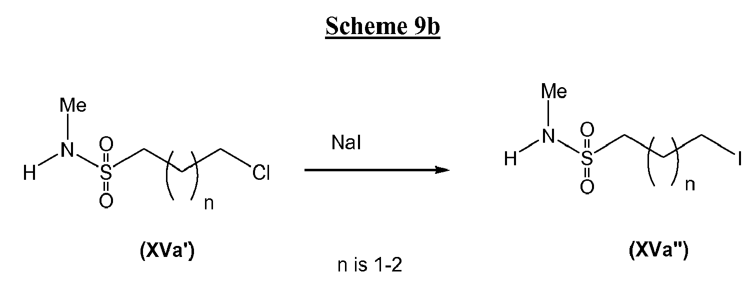

In another example, when Y is C₅alkyl (i.e. pentylene), the tosylate (XVb") can be prepared in three steps from the nitrile (XVe), as is shown in Scheme 9c. In a first step the nitrile (XVe) is hydrolyzed to the carboxylic acid (XVc"). This can be effected by heating in a mixture of acetic acid and hydrochloric acid, preferably at reflux temperature. In a second step the carboxylic acid function in (XVc") is reduced to the corresponding alcohol of the formula (XVd"). This can be effected by treatment with borane in a polar aprotic solvent, such as THF or the like, at a temperature between 0 and 20°C. In a third step the hydroxyl function in (XVd") is functionalized into a tosylate group to afford (XVb"), by reacting the alcohol (XVd") with tosyl chloride in the presence of a tertiary amine, such as triethyl amine, or the like, in an apolar solvent, such as dichloromethane or the like, at room temperature.

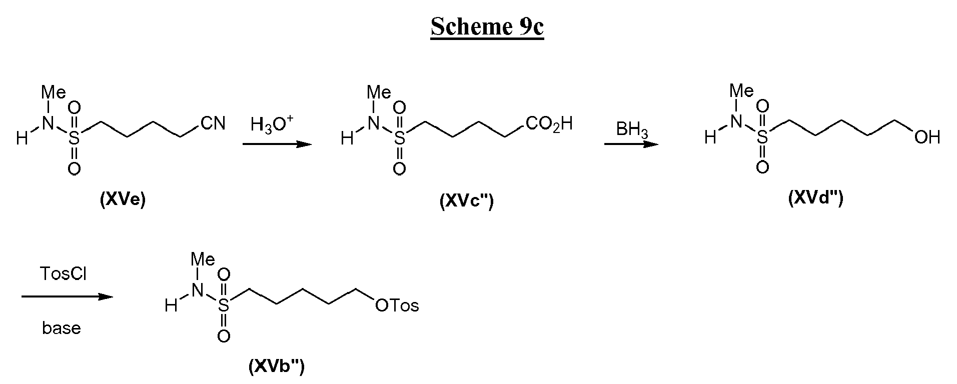

The linker precursors of the general formula (XVm) and (XVn) can be prepared as is outlined in Scheme 9d, starting from a bromo alcohol of the general formula (XVh). In a first step the alcohol in (XVh) is protected as a carboxylic ester of the general formula (XVi). Said alcohol (XVh) is treated with an acyl chloride, such as acetyl chloride or pivaloyl chloride or the like, in the presence of a tertiary amine base, such as triethyl amine, or the like, in a halogenated solvent, such as dichloro methane, or the like. The reaction can be carried out between 0°C and room temperature. In the second step, the bromo ester of the general formula (XVi) is converted into the corresponding (amino iminomethyl)thio ether of the formula (XVj). This transformation is effected by heating a mixture of thiourea and the bromoester (XVi) in a protic solvent, such as ethanol or the like, at a temperature between 70 and 100°C. In a third step the sulfonyl chloride of the general formula (XVk) is prepared by treating the (amino iminomethyl)thio ether of the formula (XVj) with chlorine in water as the solvent at a temperature of 0°C. In a fourth step the sulfonyl chloride of the general formula (XVk) is converted into the corresponding methyl sulfonamide of the general formula (XVm) by treating a mixture of the sulfonyl chloride (XVk) with methyl amine HCl salt in a biphasic solvent system consisting of water and a halogenated hydrocarbon, such as dichloro methane, or the like. Said transformation is carried out in the presence of an inorganic base, such as potassium carbonate, or the like, at a temperature between 10 and 20°C.

In a fifth step the ester protecting group in the methyl sulfonamide of the formula (XVm) is removed by treatment with a metal hydroxide, such as sodium hydroxide. The reaction is performed in an aqueous environment, and is most advantageously carried out in the presence of at least one water miscible organic co-solvent, such as methanol, ethanol or THF, or the like to afford the methyl sulfonamide alcohol of the general formula (XVn).

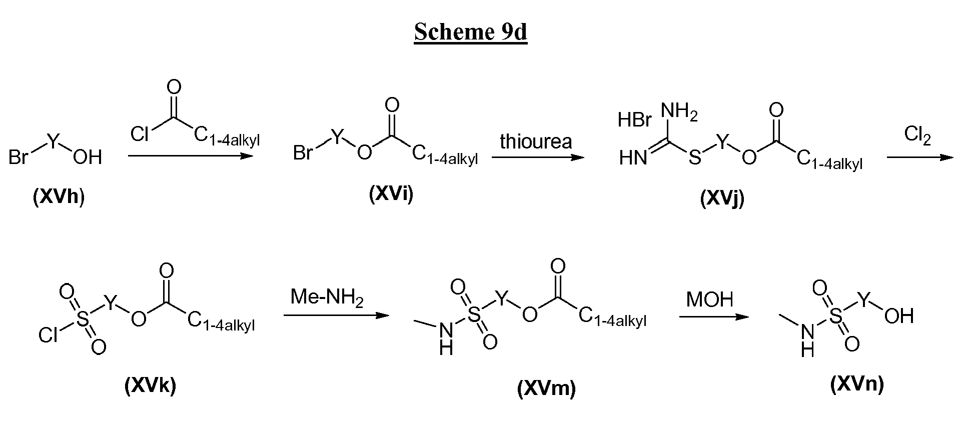

Feline animals as used throughout this text include animals from the *Felidae* family such as the domestic cat but also wild cats including the big cats such as tiger, lion, jaguar, leopard,cougar, cheetah, serval, lynxes and ocelot.

In view of the anti-FIV activity of the compounds of formula (I) as demonstrated in part B of the Working Examples, it follows that the present invention provides compounds of formula (I) for use in the treatment or prevention of feline immunodeficiency virus (FIV). Also provided is the use of a compound of formula (I) for the manufacture of a medicament for the treatment or prevention of feline immunodeficiency virus (FIV).

Furthermore the present invention also provides a method of treating or preventing infection of feline immunodeficiency virus in a feline animal wherein said method comprises the administration to said feline animal of a therapeutically effective amount of a compound of formula (I).

The term "therapeutically effective amount of a compound of formula (I)" as used herein, means that amount of compound of formula (I) that elicits the biological or medicinal response in the feline animal that is being sought by the veterinarian, which includes alleviation of the symptoms of the condition being treated. The therapeutically effective amount can be determined using routine optimization techniques and is dependent upon the particular condition to be treated, the condition of the feline animal, the route of administration, the formulation, and the judgment of the practitioner and other factors evident to those skilled in the art. A therapeutically effective amount may be achieved by multiple dosing.

Additionally the present invention provides pharmaceutical compositions comprising at least one pharmaceutically acceptable carrier and a therapeutically effective amount of a compound of formula (I).

For use in feline animals, the compounds of formula (I) can be administered alone, but will generally be administered in a mixture with a pharmaceutically or veterinary acceptable diluent or carrier selected with regard to the intended route of administration and standard pharmaceutical practice. For example, they can be administered orally, including sublingually, in the form of tablets containing such excipients as starch or lactose, or in capsules or ovules either alone or in admixture with excipients, or in the form of elixirs, solutions or suspensions containing flavouring or colouring agents. The compounds of formula (I) could be incorporated into capsules, tablets or boluses for targeting the colon or duodenum via delayed dissolution of said capsules, tablets or boluses for a particular time following oral administration. The compounds of formula (I) can be injected parenterally, for example, intravenously, intramuscularly or subcutaneously. For parenteral administration, they are best used in the form of a sterile aqueous solution or suspension that may contain other substances, for example, enough salt or glucose to make the solution isotonic with blood. The compounds of formula (I) can be administered topically, in the form of sterile creams, gels, pour-on or spot-on formulations, suspensions, lotions, ointments, dusting powders, sprays, drug-incorporated dressings or via a skin patch. For example the compounds of formula (I) can be incorporated into a cream consisting of an aqueous or oily emulsion of polyethylene glycols or liquid paraffin, or they can be incorporated into an ointment consisting of a white wax soft paraffin base, or as hydrogel with cellulose or polyacrylate derivatives or other viscosity modifiers, or as a dry powder or liquid spray or aerosol with butane/propane, HFA or CFC propellants, or as a drug-incorporated dressing either as a tulle dressing, with white soft paraffin or polyethylene glycols impregnated gauze dressings or with hydrogel, hydrocolloid, alginate or film dressings. The compounds of formula (I) could also be administered intra-ocularly as an eye drop with appropriate buffers, viscosity modifiers (e.g. cellulose derivatives), preservatives (e.g. benzalkonium chloride (BZK)) and agents to adjust tenicity (e.g. sodium chloride). Such formulation techniques are well-known in the art. All such formulations may also contain appropriate stabilizers and preservatives.

For veterinary use, compounds can be administered as a suitably acceptable formulation in accordance with normal veterinary practice and the veterinarian will determine the dosing regimen and route of administration which will be most appropriate for a particular animal.

For topical application dip, spray, powder, dust, pour- on, spot-on, emulsifiable concentrate, jetting fluid, shampoos, collar, tag or harness may be used. Such formulations are prepared in a conventional manner in accordance with standard veterinary and pharmaceutical practice. Thus capsules, boluses or tablets may be prepared by mixing the active ingredient with a suitable finely divided diluent or carrier, additionally containing a disintegrating agent and/or binder such as starch, lactose, talc, or magnesium stearate. A drench formulation may be prepared by dispersing the active ingredients in an aqueous solution together with dispersing or wetting agents and injectable formulations may be prepared in the form of a sterile solution or emulsion. Pour-on or spot-on formulations may be prepared by dissolving the active ingredients in an acceptable liquid carrier vehicle, such as butyl digol, liquid paraffin or non-volatile ester with or without addition of a volatile component such as isopropanol.

Alternatively, pour-on, spot-on or spray formulations can be prepared by encapsulation to leave a residue of active agent on the surface of the animal. These formulations will vary with regard to the weight of active compound depending on the species of host animal to be treated, the severity and type of infection and type and body weight of the host. The formulations comprising a compound of formula (I) may be administered continuously, particularly for prophylaxis by known methods.

As an alternative the combinations may be administered with the animal feedstuff and for this purpose a concentrated feed additive or premix may be prepared for mixing with the normal animal feed.

Those skilled in the treatment of feline immunodeficiency virus will easily determine the therapeutically effective amount of a compound of formula (I) from the test results presented hereinafter. In general it is contemplated that a therapeutically effective dose will be from about 0.1 mg/kg to about 100 mg/kg of body weight, more preferably from about 1 mg/kg to about 10 mg/kg of body weight of the feline animal to be treated.

The exact dosage and frequency of administration depends on the particular compound of formula (I) used, the particular condition being treated, the severity of the condition being treated, the age, weight and general physical condition of the particular feline animal as well as the other medication, the feline animal may be taking, as is well known to those skilled in the art. Furthermore, said therapeutically effective amount may be lowered or increased depending on the response of the treated animal and/or depending on the evaluation of the physician or veterinarian prescribing the compounds of the instant invention. The frequency of administration can be once daily or several times throughout the day but a frequency of once weekly or once monthly is also a possibility.

The disclosure also extends to a product comprising a compound of formula (I) and an anti-viral agent as a combined preparation for simultaneous, separate or sequential use in the treatment or prophylaxis of feline immunodeficiency virus infection. Co-administration as employed herein where the active agents are provided as separate formulations and administered either approximately simultaneously (at about the same time) or sequentially. Sequential administration is intended to refer to where one active is still present in the patient, when the second or third active is administered. Said other antiviral compounds may be any known antiretroviral compounds such as nucleoside reverse transcriptase inhibitors (NRTIs), e.g. zidovudine (AZT), didanosine (ddI), zalcitabine (ddC), lamivudine (3TC), stavudine (d4T), emtricitabine (FTC), abacavir (ABC), amdoxovir (DAPD), elvucitabine (ACH-126,443), apricitabine (AVX 754, (-)-dOTC), fozalvudine tidoxil (FZT, HDP-990003), phosphazide, KP-1461, racivir (PSI-5004), MIV-210, and GS-9131; non-nucleoside reverse transcriptase inhibitors (NNRTIs) such as delavirdine (DLV), efavirenz (EFV), nevirapine (NVP), dapivirine (TMC120), etravirine (ETR, TMC125), rilpivirine (TMC278), IDX899, RDEA-806, UK-453601, RDEA-427, and UC-781; nucleotide reverse transcriptase inhibitors (NtRTIs), e.g. tenofovir and its pro-drug tenofovir disoproxil fumarate (TDF); protease inhibitors, e.g. ritonavir (RTV), saquinavir (SQV), lopinavir (ABT-378, LPV), indinavir (IDV), amprenavir (VX-478), nelfinavir (AG-1343), atazanavir (BMS 232,632), darunavir (TMC114), fosamprenavir (GW433908 or VX-175), brecanavir (GW-640385, VX-385), tipranavir (PNU-140690), DG-17, SPI256, PPL-100 (MK 8122), and TMC310911; entry inhibitors, which comprise fusion inhibitors (e.g. enfuvirtide (T-20) sifuvirtide, HRG-214, albuvirtide, SUC-HAS, and maC46/M87o), attachment inhibitors, modulators of intracellular cholesterol and corticosteroid biosynthesis (e.g. SP-01A), and co-receptor inhibitors, the latter comprise the CCR5 antagonists (e.g. CCR5mAb004, maraviroc (UK-427,857), PRO-140, TAK-220, TAK-652, PF232798, vicriviroc (SCH-D, SCH-417,690), GSK-706769, nifeviroc, and SCH-532706) and CXR4 antagonists (e.g. AMD-070), further examples of entry inhibitors are TNX-355, INCB 9471, BMS-488043, nonakine, and VGV-1; maturation inhibitors, e.g. bevirimat (PA-457) and vivecon; and inhibitors of the viral integrase, e.g. raltegravir (MK-0518), elvitegravir (JTK-303, GS-9137), BMS-538158, S-349572, JTK-656 S-247303, and GS-265744.

### WORKING EXAMPLES

### A. Chemical synthesis of compounds of formula (I)

### Example 1 - Methyl 8-(benzyloxy)-5-bromo-1,6-naphthyridine-7-carboxylate

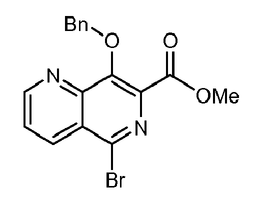

Benzyl bromide (2.53 ml, 21.2 mmol) was added to a mixture of methyl 5-bromo-8-hydroxy-1,6-naphthyridine-7-carboxylate (3.0 g; 10.6 mmol) and cesium carbonate (6.9 g; 21.2 mmol) in DMF (30 ml), under stirring at room temperature. The reaction mixture was stirred at room temperature for 12 hours. Water and ethyl acetate were added. The organic phase was separated, washed with water and with a saturated NaCl solution. The organic phase was dried, filtered and concentrated to dryness. The crude material was purified by flash chromatography column over silica gel (eluting with a gradient hexane-ethyl acetate 10:1 to 2:1).
The product fractions were collected and the solvent was evaporated.
Yield: 3.3 g (83%).

### Example 2 - ethyl 2-cyano-5-fluorobenzoate

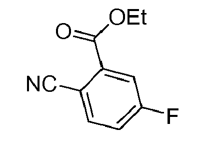

Zinc cyanide (26.5 g; 0.225 mol) and Pd2(dba)3 (1.9 g; 3.4 mmol) were added to a solution of ethyl 2-bromo-5-fluorobenzoate (27.9 g; 0.112 mol) in DMF (71 ml). Triphenyl phosphine (2.9 g; 11 mmol) was added and then the reaction mixture was stirred at 130°C under nitrogen atmosphere for 4 hours. The mixture was poured out into H₂O (300 ml) and then extracted with EtOAc (200 ml). The organic layer was separated, washed with H₂O (2 x 100 ml), brine (100 ml), dried (MgSO₄), filtered and the solvent was evaporated.
The residue was purified by column chromatography over silica gel (eluent: hexanes/EtOAc (5/1, v/v)). The product fractions were collected and the solvent was evaporated.
Yield: 20 g (93%; orange solid).

### Example 3 - Ethyl 2-((tert-butoxycarbonylamino)methyl)-5-fluorobenzoate

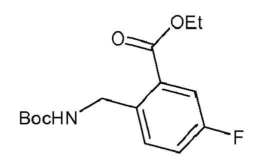

A mixture of ethyl 2-cyano-5-fluorobenzoate (Example 2; 20 g; 0.10 mol), Boc₂O (24 ml; 0.11 mol), sodium bicarbonate (9.4 g; 0.11 mol) and Raney nickel (2 g) in THF (414 ml) was hydrogenated under a 3 bar pressure of H2 at 50 ° C for 24 hours. After uptake of H2, the catalyst was removed by filtration over a Celite path and the Celite was washed with THF. The filtrate was evaporated. The residue was purified by column chromatography over silica gel (eluent: hexanes/EtOAc (5/1, v/v). The product fractions were collected and the solvent was evaporated. Yield: 15.9 g (52 %)

### Example 4 - Ethyl 2-(aminomethyl)-5-fluorobenzoate hydrochloride

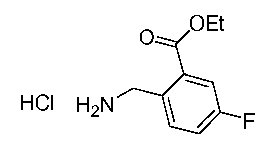

Concentrated HCl (37%; 16 ml) was added dropwise to a solution of thyl 2-((tert-butoxycarbonylamino)methyl)-5-fluorobenzoate (Example 3; 15.9 g; 53.6 mmol) in THF (96 ml). The mixture was heated at 50°C for 2 hours.The mixture was concentrated to dryness.The residue was taken up in ethanol (200 ml) and concentrated again. The solid was triturated from ether (100 ml). The precipitate was filtered off and washed with diethyl ether (2 x 50 ml).
Yield: 8.5 g (81%; as a colourless solid).

### Example 5 - 4-chloro-N-methylbutane-1-sulfonamide

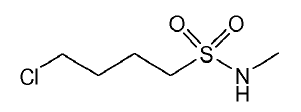

To a solution of 4-chlorobutane-l-sulfonyl chloride (117 mmol) and triethyl amine (117 mmol) in dichloro methane (250 ml) at 0°C was added a 2M solution of methyl amine in THF (117 mmol) dropwise. The mixture was allowed to warm to room temperature with stirring overnight. The organic solution was washed with H₂O and extracted with CH₂Cl₂, dried over MgSO₄ and filtered. The solution was concentrated. The product was used without further purification.

### Example 6 - 4-azido-N-methylbutane-1-sulfonamide

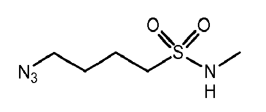

A solution of 4-chloro-N-methylbutane-1-sulfonamide (Example 5; 180 mmol), and sodium iodide (198 mmol) in DMF (180 ml) was stirred for 10 min at room temperature. Sodium azide (397 mmol) was added and the reaction mixture was stirred overnight at 60°C. The mixture was filtered off. The organic phase was extracted with AcOEt/water. The crude mixturewas concentrated and was used without further purification in the next step.

### Example 7 - 4-amino-N-methylbutane-1-sulfonamide

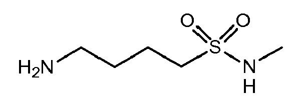

To a solution of 4-azido-N-methylbutane-1-sulfonamide (Example 6; 202 mmol), in methanol (200 ml) was added Pd/C (20 mmol Pd). The mixture put under an hydrogen atmosphere and was stirred overnight at r.t. The mixture was filtered and concentrated. The crude product was used in the next step without further purification.

### Example 8 - tert-butyl 4-(N-methylsulfamoyl)butylcarbamate

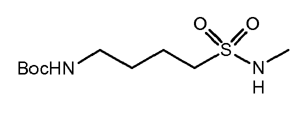

To a solution of 4-amino-N-methylbutane-1-sulfonamide (Example 7; 183 mmol), in dichloro methane (350 ml) was added portion wise Boc₂O (183 mmol). The mixture was stirred overnight at r.t. The crude product was concentrated and purified by column chromatography (CH₂Cl₂:MeOH 100:1) to give the target material in 62% yield.

### Example 9 - 4-Cyano-N-methylbutane-1-sulfonamide

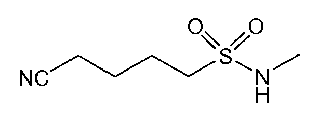

A solution of 4-chloro-N-methylbutane-1-sulfonamide (Example 5; 86.7 mmol), and sodium iodide (95.4 mmol) in DMF (175 ml) was stirred for 10 min at room temperature. Sodium cyanide (191 mmol) was added and the mixture was stirred overnight at 60°C. The mixture was filtered off and the filtrate was extracted with AcOEt/water. The crude product was concentrated and was used without further purification in the next step (Examples 10 and 20).

### Example 10 - 5-Amino-N-methylpentane-1-sulfonamide

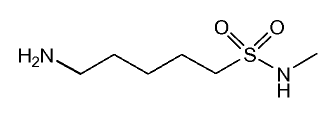

A mixture of 4-cyano-N-methylbutane-1-sulfonamide (Example 9; 91.2 mmol) and Pd/C (10 mol%, 1 g) in methanol/7N NH₃ (180 ml) was put under a hydrogen atmosphere. The mixture was stirred overnight at room temperature. The mixture was filtered and concentrated. The crude product was used in the next step without further purification.

### Example 11 - tert-Butyl 5-(N-methylsulfamoyl)pentylcarbamate

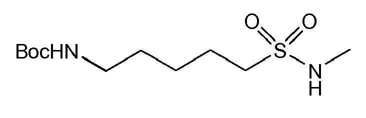

To a solution of 5-Amino-N-methylpentane-1-sulfonamide (Example 10; 42.6 mmol), in dichloro methane (92 ml) was added portion wise Boc₂O (42.6 mmol). The mixture was stirred overnight at room temperature. The crude product was concentrated and purified by column chromatography (CH₂Cl₂:MeOH 100:1) to give the target material in 50% yield.

### Example 12 - Methyl 5-bromo-8-(tosyloxy)-1,6-naphthyridine-7-carboxylate

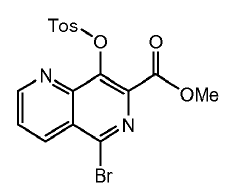

Triethyl amine (15.9 mmol) was added to a suspension of methyl 5-bromo-8-hydroxy-1,6-naphthyridine-7-carboxylate (10.6 mmol) in chloroform (22 ml) over 5 min. at 20-50°C. Tosyl chloride (12.7 mmol) was added over 5 min maintaining the temperature at 40°C for 2 h. The mixture was cooled to 20°C over 15 min. MeOH was added over 30 min, then a mixture of MeOH:water was added over 30 min. The resulting off-white crystalline solid was collected by filtration and dried to give the target compound in 50% yield.

### Example 13 - Methyl 8-(benzyloxy)-5-(piperazin-1-yl)-1,6-naphthyridine-7-carboxylate

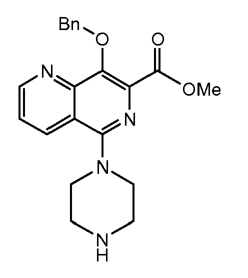

Diisopropyl ethyl amine (42.9 mmol) was added to a solution of methyl 8-(benzyloxy)-5-bromo-1,6-naphthyridine-7-carboxylate (Example 1; 10.7 mmol) in DMA (270 ml) at room temperature. The reaction was stirred at 5 min at. Piperazine (16.1 mmol) was added and the reaction mixture was heated at 110°C for 12 hr.
The reaction was cooled, water and ethyl acetate was added. The organic layer was separated and washed with water (x2) and with brine. The organic layer was dried (MgSO₄), filtered and evaporated.
The residue was purified by flash-chromatography on Si02 with (Hex/AcOEt 10:1-1: 1), to give the title compound (1.5 g) as a pale yellow solid.

### Example 14 - Tert-butyl 2-(benzyloxy)-4-fluorobenzylcarbamate

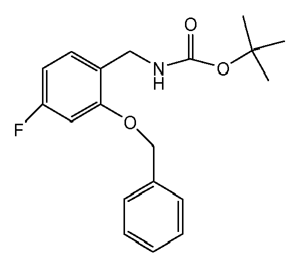

(2-(Benzyloxy)-4-fluorophenyl)methanamine was dissolved in 1,4-dioxane (22 ml). Water (6 ml) and 1M Na₂CO₃ solution (55 ml) were added. The reaction mixture was cooled to 0°C. Boc₂O was added dropwise. The ensuing mixture was allowed to warm to r.t. and stirred at r.t. for 24 hours.
The reaction mixture was filtered and washed with CH₂Cl₂ and water (x2). The phases were separated and the organic layer was dried with MgSO₄, filtered and concentrated to afford the crude target compound as a yellow solid (5.75 g). This material was combined with batches from separate experiments, and further purified by column chromatography over SiO₂ (Hexanes/AcOEt, 8/1, v/v) to afford the target compound (97% purity) as a colorless solid.

### Example 15 - Tert-butyl4-fluoro-2-hydroxybenzylcarbamate

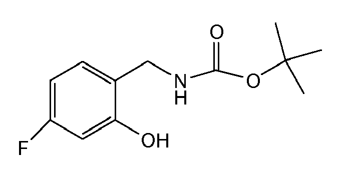

A solution of tert-butyl 2-(benzyloxy)-4-fluorobenzylcarbamate (Example 14; 9 mmol) in EtOH (80 ml) and ethyl acetate (240 ml) was treated with 1 atm of hydrogen at 25°C over 10 % Pd/C (0.4 gr) for 24 hours. The catalyst was removed by filtration through celite, washed with EtOH and the filtrate was concentrated to afford the title compound (2.2 g) as a yellow solid.

### Example 16 - Ethyl 5-(2-((tert-butoxycarbonylamino)methyl)-5-fluorophenoxy)-pentanoate

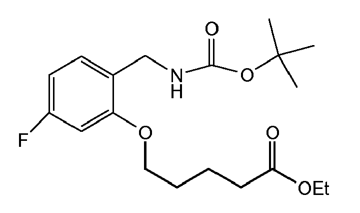

Ethyl 5-bromopentanoate (14.9 mmol) was added dropwise over 15 minutes to a mixture of tert-butyl 4-fluoro-2-hydroxybenzylcarbamate (Example 15; 12.4 mmol) and potassium carbonate (16.1 mmol) in DMA (37 ml). The reaction was heated at 60°C for 4 hours, followed by the addition of another amount of ethyl 5-bromopentanoate (4.5 mmol) and potassium carbonate (4.8 mmol) and heating at 60°C for 2 hours. The solvent was evaporated. The residue was taken up in AcOEt and filtered. The filtrate was washed with AcOEt (x3) and concentrated in vacuo. The residue was purified by column chromategraphy over Si02 (Hexanes/AcOEt, 8/1, v/v) to afford the target compound (4.1 gr, 96% purity).

### Example 17 - 5-(2-((Tert-butoxycarbonylamino)methyl)-5-fluorophenoxy)pentanoic acid

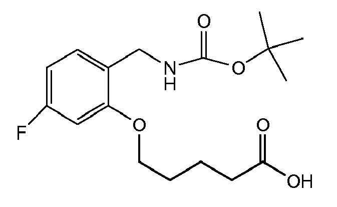

Ethyl 5-(2-((tert-butoxycarbonylamino)methyl)-5-fluorophenoxy)pentanoate (Example 16; 1.6 mmol) was dissolved in a mixture of THF (6 ml), methanol (6 ml) and water (6 ml). Sodium hydroxide (5.0 mmol) was added and stirred for 2 h. The organic solvent was removed under vacuum, and the aqueous residue was acidified with 1 N HCl to pH= 3. The mixture was extracted with ethyl acetate, and the organic phase was removed under vacuum to afford the title compound (0.55 gr) that was used as such in the next step (Example 5.1).

### Example 18 - 4-Iodo-N-methylbutane-1-sulfonamide

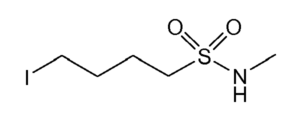

in acetone (600 ml) was stirred at refluxed overnight. The mixture was allowed to cool to room temperature and filtered. The filtrate was concentrated in vacuo to afford the title compound (40 g), that was used as such in the next step (Example 19).

### Example 19 - Tert-butyl 4-fluoro-2-(4-(N-methylsulfamoyl)butoxy)benzylcarbamate

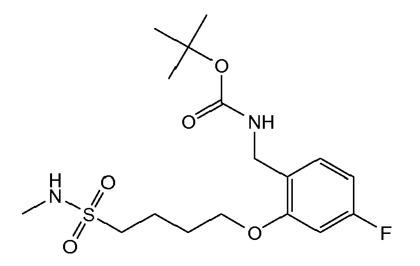

A mixture of tert-butyl 4-fluoro-2-hydroxybenzylcarbamate (Example 15; 12.2 mmol), 4-iodo-N-methylbutane-1-sulfonamide (Example 18; 18.3 mmol) and potassium carbonate (61 mmol) in DMF (60 ml) was stirred at 80°C for 10 hours.
The reaction mixture was filtered off and the residue was washed with ethyl acetate. The filtrate was concentrated and water was added.
The mixture was extracted with ether and the organic layer was separated, dried over MgSO₄, filtered off and concentrated to give the crude product. The crude product was combined with another batch from a separate experiment and then purified by RP preparative high-performance liquid chromatography (eluent: MeOH/ H₂O from 50/ 50 to 80/ 20, 0.1 % CF3COOH).
The pure fractions were collected and saturated NaHCO₃ solution was added.
The solvent was concentrated under vacuum and then extracted with dichloromethane. The organic layer was dried over MgSO₄, filtered off and concentrated to give the target compound (1.1 gr, 13% yield).

### Example 20 - 5-(N-Methylsulfamoyl)pentanoic acid

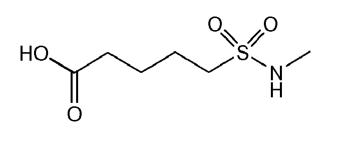

A mixture of 4-cyano-N-methylbutane-l-sulfonamide (Example 9; 100 mmol), acetic acid (100 ml) and concentrated hydrochloric acid (100 ml) was refluxed for 5 hours and then concentrated in vacuo. The residue was taken on THF and then filtered off. The filtrate was concentrated and the residue was washed with dichloromethane to give the pure product (11 g; 58% yield).

### Example 21 - 5-Hydroxy-N-methylpentane-1-sulfonamide

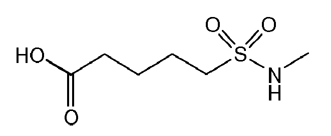

To a cooled solution of 5-(N-methylsulfamoyl)pentanoic acid (Example 20; 26 mmol) in THF (50 ml) was added a solution of borane in THF (77 mmol) at 0°C.
The reaction mixture was allowed to warm to room temperature and refluxed for 3 hours. Methanol was added and the mixture was concentrated in vacuo to give the crude product. The crude product was purified by flash column chromatography (SiO₂; eluent: petroleum ether/ethyl acetate from 100/ 0 to 20/ 80) to give the pure title compound (1.2 gr; 26% yield).

### Example 22 - 5-(N-Methylsulfamoyl)pentyl 4-methylbenzenesulfonate

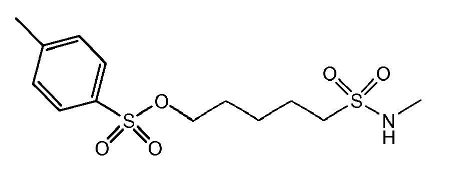

To a solution of 5-hydroxy-N-methylpentane-1-sulfonamide (Example 21; 6.4 mmol) and triethyl amine (19.2 mmol) in THF (15 ml) was added *para* toluenesulfonyl chloride (5.9 mmol) in THF (5 ml) at room temperature and the reaction mixture was stirred for 10 hours. The reaction mixture was washed by saturated NaHCO₃ solution and then brine. The organic layer was dried over MgSO₄, filtered off and concentrated.
The residue was purified by flash column chromatography (SiO₂; eluent: petroleum ether/ethyl acetate from 100/ 0 to 50/ 50) to give the pure title compound (0.9 gr; 41% yield.

### Example 23 - Tert-butyl 4-fluoro-2-(5-(N-methylsulfamoyl)pentyloxy)benzylcarbamate

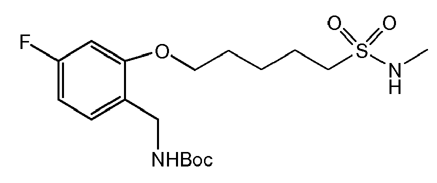

A mixture of 5-(N-methylsulfamoyl)pentyl 4-methylbenzenesulfonate (Example 22; 2.6 mmol), tert-butyl 4-fluoro-2-hydroxybenzylcarbamate (Example 15; 2.1 mmol) and potassium carbonate (6.3 mmol) in DMSO (20 ml) was stirred at 50°C under nitrogen atmosphere for 2 hours. The reaction mixture was taken on in dichloromethane and filtered off. The filtrate was washed with saturated NaHCO₃ solution and brine. The organic layer was concentrated in vacuo and then methyl t-butyl ether was added.
The mixture was washed with brine and the organic layer was dried over MgSO₄, filtered off and concentrated in vacuo to give the crude product.
The crude product was purified by flash column chromatography (SiO₂; eluent: petroleum ether/ethyl acetate from 100/ 0 to 50/ 50) to give the pure title compound (0.7 gr; 82% yield).

### Example 1.1 - Methyl 8-(benzyloxy)-5-(6-(tert-butoxycarbonylamino)hexylamino)-1,6-naphthyridine-7-carboxylate

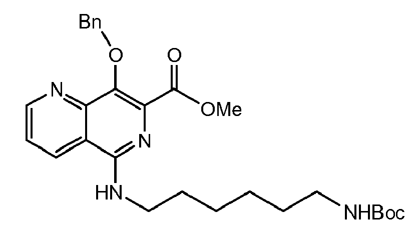

Methyl 8-(benzyloxy)-5-bromo-1,6-naphthyridine-7-carboxylate (Example 1; 5.50 g; 14.7 mmol) was dissolved in DMA (300 ml). DIPEA (5.14 ml; 29.5 mmol) was added at room temperature. The reaction mixture was stirred for 5 min at room temperature. tert-Butyl 6-aminohexylcarbamate (4.95 ml; 22.1 mmol) was added and the reaction mixture was stirred at 100°C for 12 hours. The reaction mixture was cooled. Water and ethyl acetate were added. The organic layer was separated and washed with water (x 2) and with brine. The organic layer was dried (MgSO₄), filtered and the solvent was evaporated. The residue was purified by flash column chromatography over silica gel (eluent: hexane/EtOAc from 10:1 to 1:1). The product fractions were collected and the solvent evaporated.
Yield: 33%; pale yellow solid

### Example 1.2 - 8-(Benzyloxy)-5-(6-(tert-butoxycarbonylamino)hexylamino)-1,6-naphthyridine-7-carboxylic acid

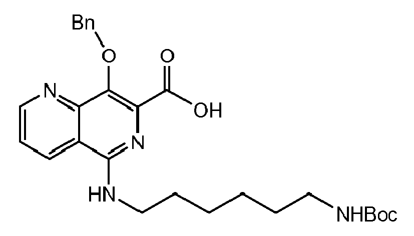

Methyl 8-(benzyloxy)-5-(6-(tert-butoxycarbonylamino)hexylamino)-1,6-naphthyridine-7-carboxylate (Example 1.1; 0.58 g; 1.08 mmol)was dissolved in a mixture of water (2 ml) and methanol (2 ml). NaOH (100 mg; 2.7 mmol) was added at room temperature. The reaction mixture was stirred at room temperature for 4 hours. H₂O and HCl 2 N were added until pH = 4-5 was reached. The mixture was extracted with EtOAc (2 x). The organic layer was separated, dried (MgSO₄), filtered and the solvent was evaporated.
Yield: 563 mg (95%).

### Example 1.3 - Ethyl 2-((8-(benzyloxy)-5-(6-(tert-butoxycarbonylamino)hexylamino)-1,6-naphthyridine-7-carboxamido)methyl)-5-fluorobenzoate

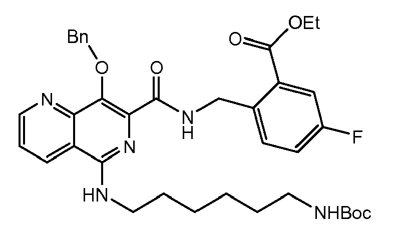

8-(B enzylo xy)-5 -(6-(tert-butoxycarbonylamino)hexylamino)-1, 6-naphthyridine-7-carboxylic acid (Example 1.2; 0.42 g; 0.85 mmol) was dissolved in dichloro methane (9 ml). DIPEA (0.58 ml; 3.42 mmol) and HBTU (0.39 g; 1.03 mmol) were added. The reaction mixture was stirred at room temperature during 5 min. Ethyl 2-(aminomethyl)-5-fluorobenzoate hydrochloride (Example 4; 0.24 g; 1.03 mmol) was added portionwise at room temperature. The mixture was stirred overnight at room temperature. The mixture was diluted with CH₂Cl₂ and washed with a saturated aqueous Na₂CO₃ solution (2 x) and H₂O. The organic layer was separated, dried (MgSO₄), filtered and evaporated.The residue was purified by flash column chromategraphy over silica gel (eluent: hexane/EtOAc 10:1 up to 1:1). The fractions were collected and the solvent evaporated.
Yield: 0.643 g

### Example 1.4 - 2-((8-(Benzyloxy)-5-(6-(tert-butoxycarbonylamino)hexylamino)-1,6-naphthyridine-7-carboxamido)methyl)-5-fluorobenzoic acid

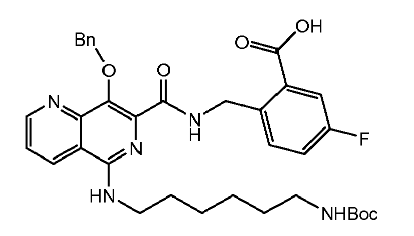

Ethyl 2-((8-(benzyloxy)-5-(6-(tert-butoxycarbonylamino)hexylamino)-1,6-naphthyridine-7-carboxamido)methyl)-5-fluorobenzoate (Example 1.3; 0.64 g; 0.96 mmol) was dissolved in ethanol (4 ml). A solution of 1N NaOH (1.5 ml) was added at room temperature. After completion of the reaction, HCl 1N was added until pH = 2-3 was reached. The solvent was evaporated under pressure. The residue was taken-up in EtOAc and washed with H₂O and brine. The organic layer was separated, dried (MgSO₄), filtered and the solvent was evaporated under reduced pressure.
Yield: 0.592 g (96%; used in next reaction step, without further purification).

### Example 1.5 - 2-((5-(6-Aminohexylamino)-8-(benzyloxy)-1,6-naphthyridine-7-carboxamido)methyl)-5-fluorobenzoic acid, TFA salt

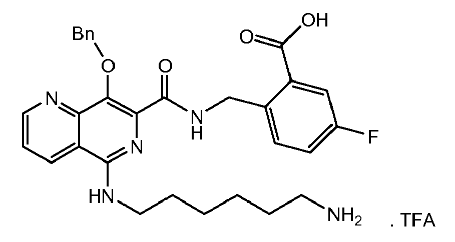

A solution of CH₂Cl₂ (3.6 ml), trifluoro acetic acid (3.6 ml) and triisopropyl silane (0.075 ml) was prepared.
2-((8-(Benzyloxy)-5-(6-(tert-butoxycarbonylamino)hexylamino)-1,6-naphthyridine-7-carboxamido)methyl)-5-fluorobenzoic acid (Example 1.4; 0.48 g; 0.74 mmol) was dissolved in dichloromethane (4 ml), and cooled to 0°C. The above prepared solution was added to this cold solution, and the reaction mixture was stirred and gradually warmed from 0°C up to room temperature during 1 hour. The solvent was evaporated under reduced pressure and the residue was distilled azeotropically with toluene (2 x). The resulting residue was dried under high-vacuum and used in the next step without further purification.
Yield: 0.420g (96%).

### Example 1.6 - Macrocyclization

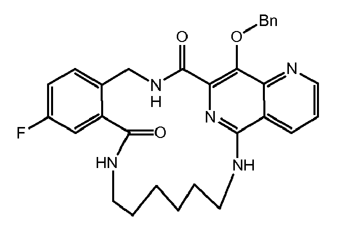

A solution of 2-((5-(6-aminohexylamino)-8-(benzyloxy)-1,6-naphthyridine-7-carboxamido)methyl)-5-fluorobenzoic acid, TFA salt (Example 1.5; 0.42 g; 0.74 mmol) in DMF (40 ml) was slowly added to a solution of HBTU (0.84 g; 2.22 mmol) and DIPEA (3.77 ml; 22.2 mmol) in DMF (150 ml) at room temperature over 4 hours. The reaction mixture was concentrated. NH40H (3 ml) was added, and the mixture was stirred for 30 min at room temperature. The solvent was evaporated till dryness.
The residue was partitioned between dichloromethane and a saturated aqueous of NaHCO₃ solution (x 2). The layers were separated. The organic layer was dried (MgSO₄), filtered and the solvent was evaporated. The crude material was purified by flash column chromatography over silica gel (eluent: CH₂Cl₂/MeOH from 80:1 up to 20:1). The product fractions were collected and the solvent was evaporated. The residue was triturated from acetonitrile, the product was collected by filtration and dried.
Yield: 0.080 g (colorless crystals).

### Example 2.1 - Methyl 5-(5-(tert-butoxycarbonylamno)-N-methylpentylsulfonamido)-8-(tosyloxy)-1,6-naphthyridine-7-carboxylate

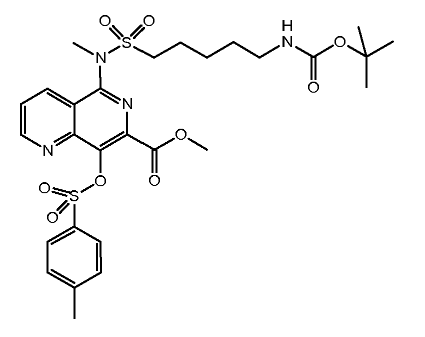

A mixture of methyl 5-bromo-8-(tosyloxy)-1,6-naphthyridine-7-carboxylate (Example 12; 10.3 mmol), tert-Butyl 5-(N-methylsulfamoyl)pentylcarbamate (Example 11; 12.4 mmol), 2,2'-bipyridine (12.4 mmol) and copper (I) oxide (12.4 mmol) in DMF (22 ml) was degassed by stirring under a stream of nitrogen for 1 min and heated to 120°C for 4 h. The brown suspension became a dark red solution with a small amount of undissolved copper (I) oxide. The mixture was diluted with chloroform, celite was added and the resulting mixture was filtered through a plug of celite. The plug was washed with chloroform and the combined filtrates were stirred vigorously with a solution of EDTA in water while nitrogen was slowly bubbled in for 30 min. The upper aqueous phase become green while the lower organic phase became yellow. The organic phase was washed with a solution of EDTA in water. The organic phases were dried over MgSO₄ and concentrated. The residue was purified by SiO₂ column chromatography ( 5:1 1 to 1:1 , hexanes:ethyl acetate) to give the target product in 70% yield.

### Example 2.2 - Methyl 5-(5-(tert-butoxycarbonylamino)-N-methylpentylsulfonamido)-8-hydroxy-1,6-naphthyridine-7-carboxylate

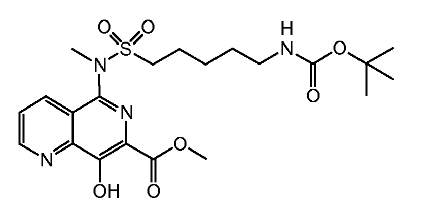

Methyl 5-(5-(tert-butoxycarbonylamino)-N-methylpentylsulfonamido)-8-(tosyloxy)-1,6-naphthyridine-7-carboxylate (Example 2.1; 8.68 mmol) was dissolved in DMF (17 ml) at 40°C and transferred to a 33% solution of NaOMe in MeOH (4.1 ml; 21.7 mmol) over 1-2 min at 25°C. The resulting yellow homogenous mixture was heated to 50°C. Mixture was cooled to 25°C over 15 min and aged at 25°C for 15 min. Acetic acid (1 ml) was added over 1 min, then water was added. The slurry was aged for 30 min and filtered. The filter cake was washed with water, and dried to give the target compound in 61 % yield.

### Example 2.3 - Methyl 8-(benzyloxy)-5-(5-(tert-butoxycarbonylamino)-N-methylpentylsulfonamido)-1,6-naphthyridine-7-carboxylate

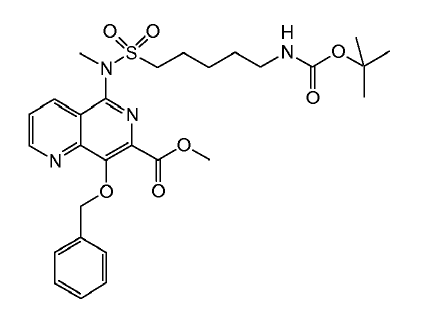

To a suspension of methyl 5-(5-(tert-butoxycarbonylamino)-N-methylpentylsulfonamido)-8-hydroxy-1,6-naphthyridine-7-carboxylate (Example 2.2; 5.44 mmol), and Cs₂CO₃ (10. 9 mmol) in DMF (11 ml) was added benzyl bromide (10.9 mmol). The mixture was stirred overnight. The crude mixture was extracted with AcOEt/water, dried over MgSO₄ and concentrated. The product was purified by SiO₂ column chromatography (1:1, AcOEt:Hex), to give the target compound in 75% yield.

### Example 2.4 - 8-(benzyloxy-5-(5-(tert-butoxycarbonylamino)-N-methylpentylsulfonamido)-1,6-naphthyridine-7-carboxylic acid

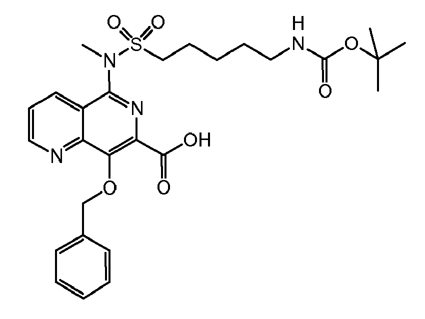

To a solution of methyl 8-(benzyloxy)-5-(5-(tert-butoxycarbonylamino)-N-methylpentylsulfonamido)-1,6-naphthyridine-7-carboxylate (Example 2.3; 4.05 mmol) in methanol (8 ml) and water (8 ml) at rt was added sodium hydroxide (16.2 mmol). The reaction was heated at 50°C. Ethyl acetate was added and the aquous phase was treated with HCl 1N until pH = 6-7. Ethyl acetate was added again, the layers were separated and the organic phase was dried with MgSO₄ and concentrated. The crude product was obtained in 94% yield, and used in the next step without further purification.

### Example 2.5 - Ethyl 2-((8-(benzyloxy)-5-(5-(tert-butoxycarbonylamino)-N-methylpentylsulfonamido)-1,6-naphthyridine-7-carboxamido)methyl)-5-fluorobenzoate

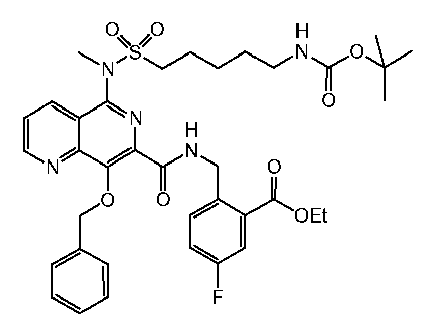

To a solution of 8-(benzyloxy)-5-(5-(tert-butoxycarbonylamino)-N-methylpentylsulfonamido)-1,6-naphthyridine-7-carboxylic acid (Example 2.4; 3.82 mmol) in dichloro methane (38 ml) were added HBTU (4.58 mmol) and DIPEA (15.8 mmol). The mixture was stirred for 5 min at rt. Ethyl 2-(aminomethyl)-5-fluorobenzoate hydrochloride (Example 4; 4.58 mmol) was added portionwise at rt. The mixture was stirred overnight at rt. The product was extracted with CH₂Cl₂, dried over MgSO₄ and concentrated. The crude product was purified by SiO₂ column chromatography (AcOEt:Hex, 1:1) to afford the target compound in 71% yield.

### Example 2.6 - 2-((5-(5-amino-N-methylpentylsulfonamido)-8-hydroxy-1,6-naphthyridine-7-carboxamido)methyl)-5-fluorobenzoic acid, HCl salt

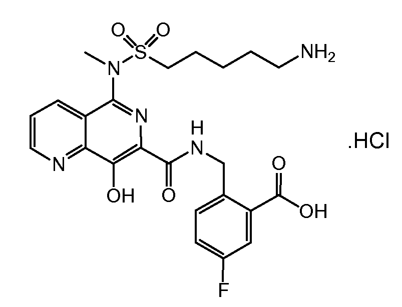

To a solution of ethyl 2-((8-(benzyloxy)-5-(5-(tert-butoxycarbonylamino)-N-methylpentylsulfonamido)-1,6-naphthyridine-7-carboxamido)methyl)-5-fluorobenzoate (Example 2.5; 2.71 mmol) in ethanol was added concentrated HCl (37%; 1.1 ml) at rt.
The reaction mixture was heated at 100°C for 3 days. The solvent was evaporated and the crude product was used in the next step without further purification.

### Example 3.1 - Methyl 5-(4-(tert-butoxycarbonylamino)-N-methylbutylsulfonamido)-8-(tosyloxy)-1,6-naphthyridine-7-carboxylate

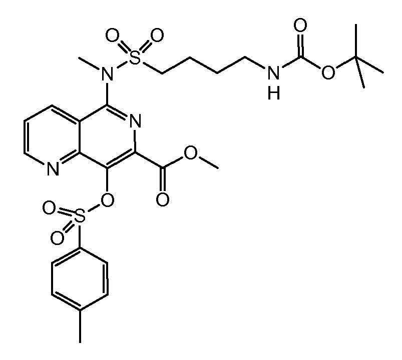

The title compound was prepared in a similar fashion as described in Example 2.1, starting
from methyl 5-bromo-8-(tosyloxy)-1,6-naphthyridine-7-carboxylate (Example 12; 10.3 mmol) and tert-butyl 4-(N-methylsulfamoyl)butylcarbamate (Example 8) to obtain the target compound (3.43 gr).

### Example 3.2 - Methyl 5-(4-amino-N-methylbutylsulfbnamido)-8-(tosyloxy)-1,6-naphthyridine-7-carboxylate

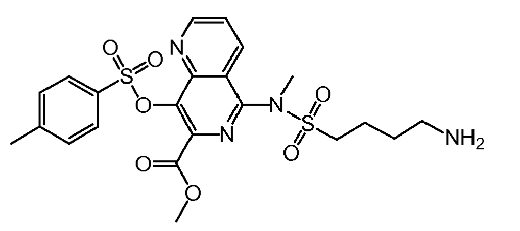

To a solution of methyl 5-(4-(tert-butoxycarbonylamino)-N-methylbutylsulfonamido)-8-(tosyloxy)-1,6-naphthyridine-7-carboxylate (Example 3.1; 5.52 mmol) in dichloro methane (5 mL) was added trifluoro acetic acid (5 mL) at 0°C. After addition the reaction was warmed to room temperature. The solvent was evaporated and the residue was co-evaporated with toluene (3x). The resulting residue was dried under vacuum and used in the next step (Example 3.3) without further purification.

### Example 3.3 - Methyl 5-(4-(2-((tert-butoxycarbonylamimo)methyl)-5-fluorobenzamido)-N-methylbutylsulfbnamido)-8-(tosyloxy)-l,6-naphthyridme-7-carboxylate

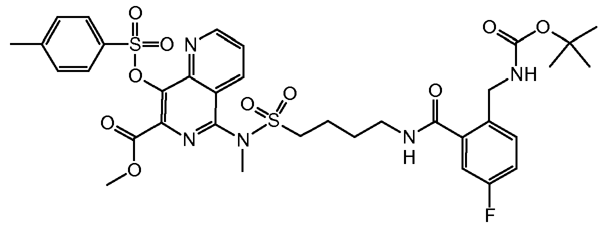

To a solution of methyl 5-(4-amino-N-methylbutylsulfonamido)-8-(tosyloxy)-1,6-naphthyridine-7-carboxylate (Example 3.2; 5.52 mmol) in dichloro methane (5 mL) was added DIPEA (18.7 mmol) and HBTU (5.63 mmol). The mixture was stirred for 5 min at rt. 2-((Tert-butoxycarbonylamino)methyl)-5-fluorobenzoic acid (4.69 mmol) was added portionwise at room temperature. The mixture was stirred overnight at room temperature. The solvent was evaporated. The residue was washed with a 1M Na₂CO₃ solution. The aqueous phase was extracted with dichloro methane, dried and was concentrated. The crude product was purified by SiO₂ column chromatography (5:1 to 1:1 hexanes:ethyl acetate) to give the target compound in 49% yield.

### Example 3.4 ― 5-(4-(2-((Tert-butoxycarbonylamino)methyl)-5-fluorobenzamido)-N-methylbutylsulfonamido)-8-(tosyloxy)-1,6-naphthyridine-7-carboxylic acid

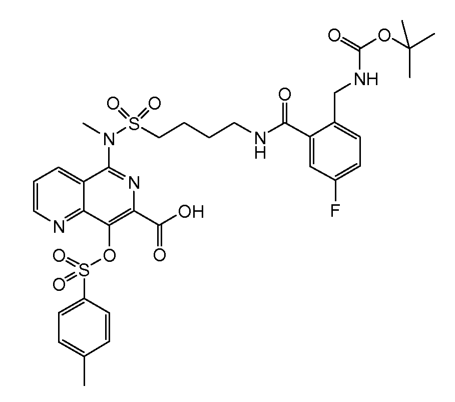

To a solution of methyl 5-(4-(2-((tert-butoxycarbonylamino)methyl)-5-fluorobenzamido)-N-methylbutylsulfonamido)-8-(tosyloxy)-1,6-naphthyridine-7-carboxylate (Example 3.3; 2.75 mmol) in a 1:1 mixture of THF and water (5.5 mL) was added lithium hydroxide (4.13 mmol) at room temperature. After the reaction was finished, ethyl acetate and water were added. The aquous layer was treated with HCl 1N until pH=5-6. The aqueous layer was extracted with ethyl acetate. The organic layer was dried over MgSO₄ and concentrated, to obtain the target material in 41 % yield.

### Example 3.5 - 5-(4-(2-(Aminomethyl)-5-fluorobenzamido)-N-methylbutylsulfonamido)-8-(tosyloxy)-1,6-naphthyridine-7-carboxylic acid, TFA salt

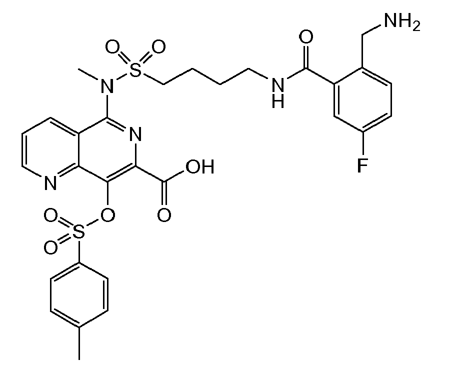

The title compound was prepared in a similar fashion as described in Example 3.2, starting from 5-(4-(2-((tert-butoxycarbonylamino)methyl)-5-fluorobenzamido)-N-methylbutylsulfonamido)-8-(tosyloxy)-1,6-naphthyridine-7-carboxylic acid (Example 3.4; 1.1 mmol).

### Example 3.6 - Macrocyclization

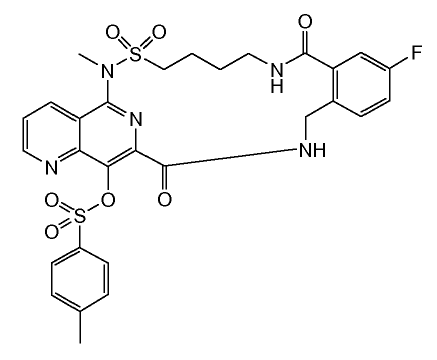

The target compound was prepared in a similar fashion as described in Example 1.6, starting from 5-(4-(2-(aminomethyl)-5-fluorobenzamido)-N-methylbutylsulfonamido)-8-(tosyloxy)-1,6-naphthyridine-7-carboxylic acid, TFA salt (Example 3.5; 1.57 mmol). The crude product was not purified and immediately used in the deprotection step (Compound 7).

### Example 4.1 - Methyl 8-(benzyloxy)-5-(4-(4-(tert-butoxycarbonylamino)butyl)-piperazin-1-yl)-1,6-naphthyridine-7-carboxylate

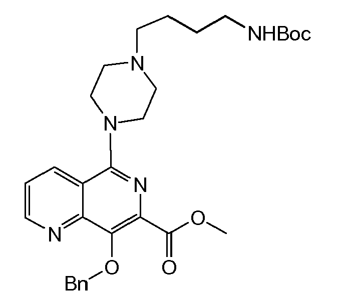

To a solution of methyl 8-(benzyloxy)-5-(piperazin-1-yl)-1,6-naphthyridine-7-carboxylate (Example 13; 1.3 mmol) in DMA (10 ml) was added potassium carbonate (2.6 mmol), followed by tert-butyl 4-bromobutylcarbamate (1.3 mmol), and the reaction was stirred at r.t for 12 hr. Water and ethyl acetate were added. The combined organic layer was washed with water, dried over MgSO₄, filtered and concentrated, to afford the crude target compound (0.89g).

### Example 4.2 - 8-(Benzyloxy)-5-(4-(4-(tert-butoxycarbonylamino)butyl)piperazin-1-yl)-1,6-naphthyridine-7-carboxylic acid

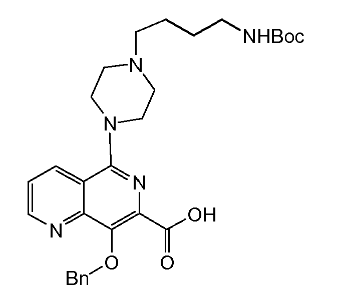

To a solution of methyl 8-(benzyloxy)-5-(4-(4-(tert-butoxycarbonylamino)butyl)-piperazin-1-yl)-1,6-naphthyridine-7-carboxylate (Example 4.1; 1.2 mmol) in a mixtire of methanol (2.5 ml) and water (2.5 ml) was added sodium hydroxide (2.5 mmol). The reaction mixture was stired at r.t. for 4 hr. The mixture was quenched with water and 2N HC1 until pH = 6 was reached.The mixture was extracted with AcOEt (2x). The organic layer was dried (MgSO₄), and filtered to afford a solid (400 mg), that was used as such in the next step.

### Example 4.3 - Ethyl 2-((8-(benzyloxy)-5-(4-(4-(tert-butoxycarbonylammo)butyl)-piperazin-1-yl)-1,6-naphthyridme-7-carboxamido)methyl)-5-fluorobenzoate

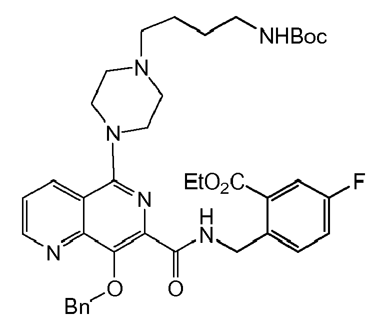

8-(Benzyloxy)-5-(4-(4-(tert-butoxycarbonylamino)butyl)piperazin-1-yl)-1,6-naphthyridine-7-carboxylic acid (Example 4.2; 1.4 mmol) was dissolved in dichloro methane (14 ml). DIPEA (5.4 mmol) and HBTU (1.6 mmol) were added. The reaction mixture was stirred at room temperature during 5 min. Ethyl 2-(aminomethyl)-5-fluorobenzoate hydrochloride (Example 4; 1.6 mmol) was added portionwise at room temperature. The mixture was stirred overnight at room temperature. The mixture was diluted with CH₂Cl₂ and washed with a saturated aqueous Na₂CO₃ solution (2 x) and H₂O. The organic layer was separated, dried (MgSO₄), filtered and evaporated.The residue was purified by flash column chromatography over silica gel (eluent: hexane/EtOAc 10:1 up to 1:1) to afford 0.97 gr of the title compound.

### Example 4.4 ― 2-((8-(Benzyloxy)-5-(4-(4-(tert-butoxycarbonylamino)butyl)piperazin-1-yl)-1.6-naphthyridine-7-carboxamido)methyl)-5-fluorobenzoic acid

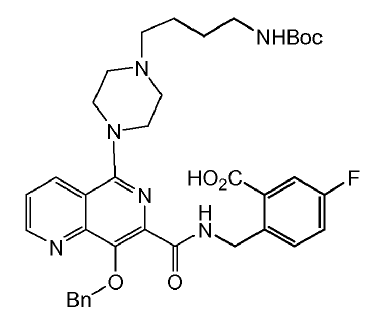

Ethyl 2-((8-(benzyloxy)-5-(4-(4-(tert-butoxycarbonylamino)butyl)piperazin-1-yl)-1,6-naphthyridine-7-carboxamido)methyl)-5-fluorobenzoate (Example 4.3; 1.4 mmol) was dissolved in THF (4.5 ml) and water (4.5 ml). Solid LiOH hydrate (1.6 mmol) was added at room temperature. The reaction mixture was stirred for 24 h, after which 2N HCl was added until pH = 6-7 was reached. The mixture was extracted twice with EtOAc and the combined organic fractions dried with magnesium sulfate. The crude material containing the title compound (0.27 gr) was used as such in the next step.

### Example 4.5 ― 2-((5-(4-(4-aminobutyl)piperazin-1-yl)-8-hydroxy-1,6-naphthyridine-7-carboxamido)methyl)-5-fluorobenzoic acid, TFA salt

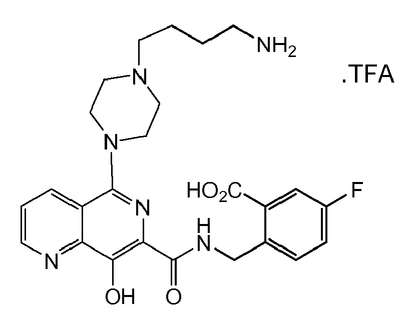

2-((8-( Benzyloxy)-5-(4-(4-(tert-butoxycarbonylamino)butyl)piperazin-1-yl)-1,6-naphthyridine-7-carboxamido)methyl)-5-fluorobenzoic acid (Example 4.4; 0.39 mmol) was dissolved in dichloromethane (1.6 ml), and cooled to 0°C. A mixture of TFA (1.6 ml) and dichloro methane (1.6 ml) was added to this cold solution, and the reaction mixture was stirred and gradually warmed from 0°C to room temperature during 1 hour. The solvent was evaporated under reduced pressure and the residue was distilled azeotropically with toluene (2 x). The resulting residue (0.42 g) was dried under high-vacuum and used in the next step (Compound 8) without further purification.

### Example 5.1 - Methyl 8-(benzyloxy)-5-(4-(5-(2-((tert-butoxycarbonylamino)methyl)-5-fluorophenoxy)pentanoyl)piperazin-1-yl)-1,6-naphthyridine-7-carboxylate

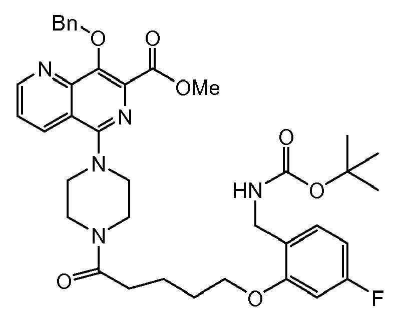

To a solution of methyl 8-(benzyloxy)-5-(piperazin-1-yl)-1,6-naphthyridine-7-carboxylate (Example 13; 1.3 mmol), 5-(2-((tert-butoxycarbonylamino)methyl)-5-fluorophenoxy)pentanoic acid (Example 17; 1.6 mmol) and diisopropyl ethylamine (3.9 mmol) in dichloromethane (10 ml) was added HBTU (2.0 mmol). The mixture was stirred overnight. The mixture was washed with saturated NaHCO₃, 10 % citric acid and brine and dried over Na₂SO₄. The solvent was removed under vacuum. The residue was purified by flash column chromatography over SiO₂ (eluent: methanol/ CH₂Cl₂, 1:100) to afford the title compound (800 mg).

### Example 5.2 - 8-(Benzyloxy)-5-(4-(5-(2-((tert-butoxycarbonylamino)methyl)-5-fluorophenoxy)pentanoyl)piperazin-l-yl)-1,6-naphthyridine-7-carboxylic acid

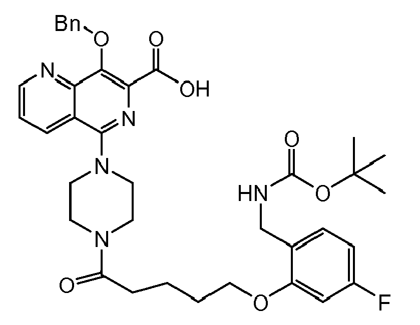

Methyl 8-(benzyloxy)-5-(4-(5-(2-((tert-butoxycarbonylamino)methyl)-5-fluorophenoxy)pentanoyl)piperazin-1-yl)-1,6-naphthyridine-7-carboxylate (Example 5.1; 1.1 mmol) was dissolved in a mixture of THF (6 ml), methanol (6 ml) and water (6 ml). Sodium hydroxide (7.5 mmol) was added and stirred for 2 days. The organic solvent was removed under vacuum, and the residue washed with diethyl ether. The aqueous residue was acidified with 1 N HCl to pH= 8-9. The mixture was washed with ethyl acetate. The aqueous phase was acidified with 1N HCl to pH 3-4. The mixture was extracted with ethyl acetate and the organic layer was washed with brine and dried over Na₂SO₄. The solvent was removed under vacuum to afford the title compound (0.4 gr) that was used as such in the next step.

### Example 5.3 - 5-(4-(5-(2-(Aminomethyl)-5-fluorophenoxy)pentanoyl)piperazin-1-yl)-8-(benzyloxy)-1,6-naphthyridine-7-carboxylic acid, TFA salt

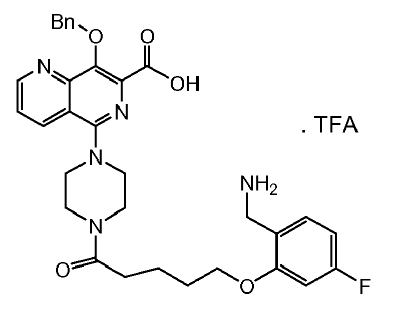

The title compound was prepared in a similar way as described in Example 1.5, from 8-(benzyloxy)-5-(4-(5-(2-((tert-butoxycarbonylamino)methyl)-5-fluorophenoxy)-pentanoyl)piperazin-1-yl)-1,6-naphthyridine-7-carboxylic acid (Example 5.2)

### Example 5.4 - Macrocyclization of 5-(4-(5-(2-(Aminomethyl)-5-fluorophenoxy)-pentanoyl)piperazin-1-yl)-8-(benzyloxy)-1,6-naphthyridine-7-carboxylic acid, TFA salt

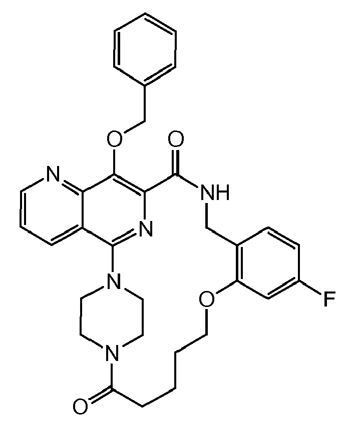

A solution of 5-(4-(5-(2-(aminomethyl)-5-fluorophenoxy)pentanoyl)piperazin-1-yl)-8-(benzyloxy)-1,6-naphthyridine-7-carboxylic acid, TFA salt (Example 5.3; 0.59 mmol) and diisopropyl ethylamine (1.8 mmol) in DMF (20 ml) was added dropwise to a mixture of pentafluorophenyl diphenylphosphinate (0.71 mmol) and diisopropyl ethylamine (0.4 ml) in diisopropyl ethylamine (1.8 mmol) in DMF (140 ml) for 30 min. The mixture was stirred for 36 h at room temperature. The solvent was removed under vacuum and the residue was dissolved in CH₂Cl₂, washed with saturated NaHCO₃, 10 % citric acid, saturated NaHCO₃ and brine and dried over Na₂SO₄. The solvent was removed under vacuum, and the solid residue was washed with ether to afford 300 mg as a powder that was used as such in the next step (Compound 10).

### Example 6.1 - Methyl 5-(4-(2-((tert-butoxycarbonylamino)methyl)-5-fluorophenoxy)-N-methylbutylsulfonamido)-8-methoxy-1,6-naphthyridine-7-carboxylate

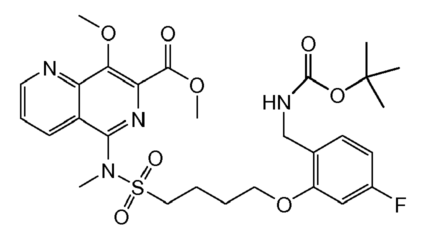

A mixture of methyl 5-bromo-8-methoxy-1,6-naphthyridine-7-carboxylate (2.4 mmol), tert-butyl 4-fluoro-2-(4-(N-methylsulfamoyl)butoxy)benzylcarbamate (Example 19; 2.6 mmol), 2,2'-bipyridine (3.1 mmol) and copper (I) oxide (3.1 mmol) in NMP (30 ml) was stirred at 120°C under nitrogen atmosphere for 10 hours. The reaction mixture was diluted with dichloromethane and filtered off. The filtrate was washed with 10 % 2-[2-(bis(carboxylatomethyl)amino)ethyl-(carboxylatomethyl)amino]acetate (EDTA) disodium salt solution, saturated NaHCO₃ solution and brine.
The organic layer was concentrated in vacuo and then methyl t-butyl ether was added. The mixture was washed with brine and the organic layer was dried over MgSO₄, filtered off and concentrated in vacuo to give the crude product.
The crude product was purified by flash column chromatography on SiO₂ (eluent: petroleum ether/ethyl acetate from 100/ 0 to 60/ 40) to give the title compound (0.48 g, 30% yield.

### Example 6.2

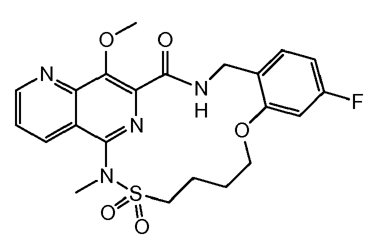

The title compound was prepared in a 3 step process from methyl 5-(4-(2-((tert-butoxycarbonylamino)methyl)-5-fluorophenoxy)-N-methylbutylsulfonamido)-8-methoxy-1,6-naphthyridine-7-carboxylate (Example 6.1) following similar procedures as described in Examples 5.2; 5.3 and 5.4.

### Example 7.1 - Methyl 5-(5-(2-((tert-butoxycarbonylamino)methyₗ)-5-fluorophenoxy)-N-methylpentylsulfonamido)-8-methoxy-1,6-naphthyridine-7-carboxylate

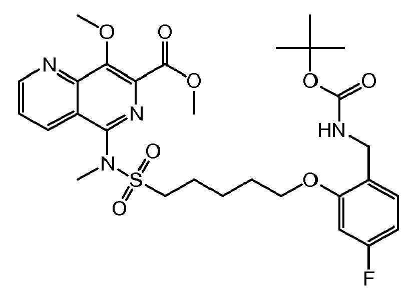

The title compound was prepared in a similar way as described for Example 6.1, using tert-butyl 4-fluoro-2-(5-(N-methylsulfamoyl)pentyloxy)benzylcarbamate (Example 23) and 5-bromo-8-methoxy-1,6-naphthyridine-7-carboxylate.

### Example 7.2

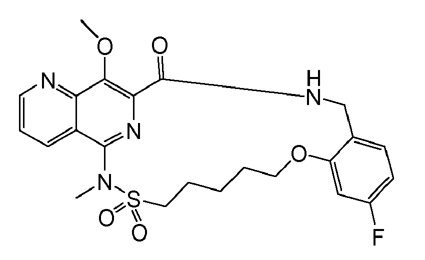

The title compound was prepared in a 3 step process from methyl 5-(5-(2-((tert-butoxycarbonylamino)methyl)-5-fluorophenoxy)-N-methylpentylsulfonamido)-8-methoxy-1,6-naphthyridine-7-carboxylate (Example 7.1) following similar procedures as described in Examples 5.2; 5.3 and 5.4.

### Compound 1

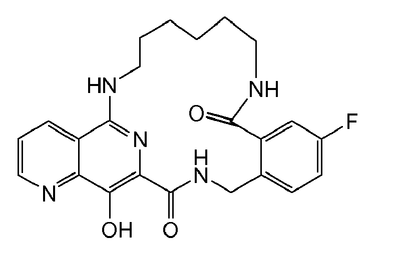

The macrocycle from Example 1.6 (80 mg; 0.15 mmol) was dissolved in 4N HCl in 1,4-dioxane (5 ml) and the mixture was heated at 40°C for 1 hour. The reaction mixture was concentrated under vacuum. The residue was triturated from methanol and the solid product was filtered off and dried.
Yield: 0.055 g (99%; HPLC purity: 96%), isolated as HCl salt
¹H NMR (400 MHz, DMSO-*d*₆) δ ppm 1.25 - 1.65 (m, 8 H) 3.44 (br. s., 4 H) 4.70 (d, *J=5.3* Hz, 2 H) 7.58 (br. s., 1 H) 7.36 (t, *J*=7.3 Hz, 1 H) 7.59 (t, *J*=6.5 Hz, 1 H) 7.64 (d, *J*=9.0 Hz, 1 H) 7.81 (br. s., 1 H) 8.73 (t, *J*=5.3 Hz, 1 H) 8.90 (d, *J*=7.2 Hz, 1 H) 9.05 (br. s., 1 H) 9.48 (t, *J*=5.3 Hz, 1 H) 12.48 (br. s., 1 H)

### Compound 2

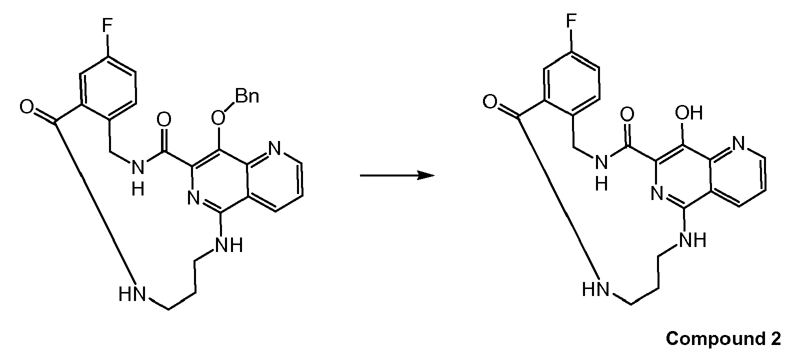

The O-benzyl protected precursor of compound 2 was prepared in an analogous fashion as described for Examples 1.1 ― 1.6, starting from methyl 8-(benzyloxy)-5-bromo-1,6-naphthyridine-7-carboxylate (Example 1) and N-tert-butoxycarbonyl-1,3-diaminopropane. De-benzylation to obtain compound 2 was carried out as follows. The O-benzyl precursor macrocycle was dissolved in dichloro methane (3.6 ml) and cooled to 0°C. To this mixture was added a freshly prepared solution, consisting of trifluoro acetic acid (3.6 ml), triisopropyl silane (0.075 ml) and dichloro methane (3.6 ml). The ensuing mixture was allowed to warm to room temperature over 1 h The reaction mixture was concentrated under vacuum. The residue was dissolved in dichloro methane, and washed with satd. NaHC0₃. The aqueous phase was extracted with dichloro methane (2x). The combined organic layers were dried (MgSO₄), filtered and the solvent was evaporated. The crude material was purified by flash column chromatography over silica gel (eluent: CH₂Cl₂/ MeOH from 50:1 up to 10:1). The product fractions were collected and the solvent was evaporated. The residue was triturated from diethyl ether, the product was collected by filtration and dried.
Yield: 0.023 g as a solid (32%; HPLC purity: 97%)
¹H NMR (400 MHz, DMSO-d₆) δ ppm 1.98 (br. s., 2 H) 3.34 (br. s., 2 H) 3.40 (br. s., 2 H) 4.78 (d, *J*=4.5 Hz, 2 H) 7.31 (t, *J*=7.5 Hz, 1 H) 7.39 (d, *J*=8.6 Hz, 1 H) 7.55 (t, *J*=6.3 Hz, 1 H) 7.69 (dd, *J*=7.2, 3.7 Hz, 1 H) 7.73 (br. s., 1 H) 8.56 - 8.72 (m, 2 H) 9.03 (d, *J*=3.7 Hz, 1 H) 9.10 (t*, J*=5.5 Hz, 1 H) 11.69 (br. s., 1 H)

### Compound 3

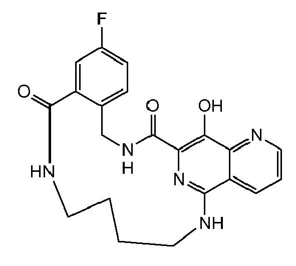

This compound was prepared in an analogous fashion as described for Examples 1.1 ― 1.6 and Compound 2, starting from methyl 8-(benzyloxy)-5-bromo-1,6-naphthyridine-7-carboxylate (Example 1) and N-tert-butoxycarbonyl-1,3-diaminobutane. The resulting compound was characterized by reversed phase HPLC using an Agilent 1100 series liquid chromatography system comprising a binary pump with degasser, an autosampler, a column oven and a UV detector, with a YMC-Pack ODS-AQ C18 column (4.6 x 50 mm). The column temperature was 35°C. The mobile phase was maintained at a flow rate of 2.6 ml/min with a gradient going from 95 % water and 5 % acetonitrile to 95 % acetonitrile in 4.80 minutes and the latter held for 1.20 minutes. Flow from the column was split to a MS spectrometer. The MS detector was configured with an electrospray ionization source. The capillary voltage was 3 kV, the quadrupole temperature was maintained at 100°C and the desolvation temperature was 300°C. Nitrogen was used as the nebulizer gas. Mass spectra were acquired by scanning from 100 to 1400. Injection volume was 10 µl. Data acquisition was performed with an Agilent Chemstation data system. Rt: 2.6 min.; MH^{+:} 410

### Compound 4

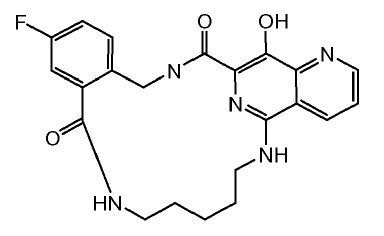

This compound was prepared in an analogous fashion as described for Examples 1.1 ― 1.6 and Compound 1, and was isolated as a HCL salt, starting from methyl 8-(benzyloxy)-5-bromo-1,6-naphthyridine-7-carboxylate (Example 1) and N-tert-butoxycarbonyl-1,3-diaminopentane.
¹H NMR (400 MHz, DMSO-d₆) δ ppm 1.45 (br. s., 4 H) 1.64 - 1.76 (qt, *J*=5.7 Hz, 2 H) 3.34 (q, *J*=5.5 Hz, 2 H) 3.71 (t, *J=5.3* Hz, 2 H) 4.80 (d, *J*=6.6 Hz, 2 H) 7.58 (br. s., 1 H) 7.32 (t, *J*=7.4 Hz, 1 H) 7.40 (d, *J*=9.2 Hz, 1 H) 7.56 (t, *J*=6.8 Hz, 1 H) 7.80 (dd, *J*=7.4, 4.3 Hz, 1 H) 8.62 (t, *J*=5.5 Hz, 1 H) 8.87 (t, *J*=6.6 Hz, 1 H) 8.91 (d, *J*=7.4 Hz, 1 H) 9.05 (d, *J*=4.3 Hz, 1 H) 12.13 (br. s., 1 H)

### Compound 5

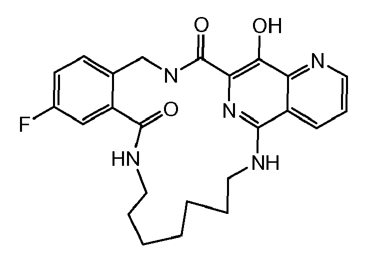

This compound was prepared in an analogous fashion as described for Examples 1.1 ― 1.6 and Compound 1, starting from methyl 8-(benzyloxy)-5-bromo-1,6-naphthyridine-7-carboxylate (Example 1) and N-tert-butoxycarbonyl-1,3-diaminoheptane. Compound 5 was isolated as HCl salt. The resulting compound was characterized by reversed phase HPLC using the method described for compound 3. Rt: 3.3 min. MH⁺: 452

### Compound 6

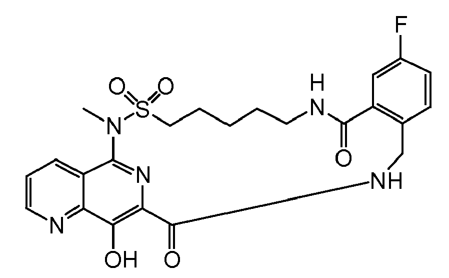

To a solution of 2-((5-(5-amino-N-methylpentylsulfonamido)-8-hydroxy-1,6-naphthyridine-7-carboxamido)methyl)-5-fluorobenzoic acid, HCl salt (Example 2.6; 1.94 mmol) in DMF (70 ml) was slowly added drop wise a solution of HBTU (5.77 mmol) and triethyl amine (58 mmol) in DMF (250 mL) at r.t. HPLC showed complete conversion. A solution of NH3 in methanol was added. The mixture was stirred for 30 min at r.t. The solvent was evaporated. The residue was purified by reverse chromatography to yield the target compound in 4% yield.
1H NMR (400 MHz, DMSO-d₆) δ ppm 1.38 (qt, *J*=6.7 Hz, 2 H), 1.61 (qt, *J*=6.5 Hz, 2 H), 1.89 (qt, *J*=7.2 Hz, 2 H), 3.31 - 3.39 (m, 4 H), 3.41 (s, 3 H), 4.74 (d, *J*=6.1 Hz, 2 H), 7.36 (td, *J*=8.6, 2.4 Hz, 1 H), 7.48 (dd, *J*=9.7, 2.1 Hz, 1 H), 7.68 (dd, *J*=8.2, 6.1 Hz, 1 H), 7.87 (dd, *J*=8.4, 4.3 Hz, 1 H), 8.58 (d, *J*=8.4 Hz, 1 H), 8.70 (t, *J*=5.5 Hz, 1 H), 9.17 (d, *J*=2.7 Hz, 1 H), 9.64 (t, *J*=6.0 Hz, 1 H), 13.65 (br. s., 1 H)

### Compound 7

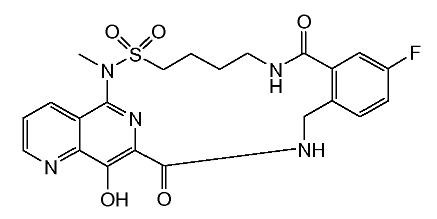

The crude macrocycle obtained in Example 3.6 (1.6 mmol) was dissolved in DMF (8 mL), and transferred to a 30% solution of NaOMe in methanol over ca 1-2 min at room temperature. The reaction mixture was quenched with acetic acid (3 mL), the solvent evaporated *in vacuo,* and the residue purified by reversed phase HPLC to afford the title compound in 14% yield over the last two steps.
1H NMR (400 MHz, DMSO-d₆) δ ppm 1.70 (quin, *J*=6.5 Hz, 2 H) 1.80 (quin, *J*=7.5 Hz, 2 H) 3.30 - 3.35 (m, 2 H) 3.37 (s, 3 H) 3.52 (br. s., 2 H) 4.74 (d, *J*=5.6 Hz, 2 H) 7.28 - 7.35 (m, 2 H) 7.62 (dd,*J*=8.1, 5.8 Hz, 1 H) 7.89 (dd,*J*=8.4, 4.1 Hz, 1 H) 8.54 (dd, *J*=8.4, 1.1 Hz, 1 H) 8.59 (t, *J*=5.2 Hz, 1 H) 8.94 (t, *J*=5.6 Hz, 1 H) 9.17 (dd, *J*=4.1, 1.1 Hz, 1 H) 13.60 (br. s., 1 H)

### Compound 8

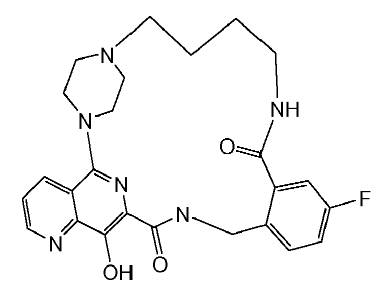

A solution of crude 2-((5-(4-(4-aminobutyl)piperazin-1-yl)-8-(hydroxy)-1,6-naphthyridine-7-carboxamido)methyl)-5-fluorobenzoic acid TFA salt (Example 4.5; 0.39 mmol) in DMF (40 ml) was slowly added to a solution of HBTU (1.2 mmol) and DIPEA (11.8 mmol) in DMF (90 ml) at room temperature over 4 hours. A 30% solution of ammonia (3 ml) was added to the reaction mixture and the volatiles removed *in vacuo.* The residue was partitioned between dichloromethane and a saturated aqueous NaHCO₃ solution (x 2). The layers were separated. The organic layer was dried (MgSO₄), filtered and the solvent was evaporated. The crude material was purified by preparative HPLC to afford the target material as a powder (56 mg).
1H NMR (400 MHz, DMSO-d₆) δ ppm 1.42 (quin, *J*=6.6 Hz, 2 H) 1.52 (quin, *J*=5.8 Hz, 2 H) 2.47 (d,*J*=11.4 Hz, 2 H) 2.70 (t,*J*=6.6 Hz, 2 H) 2.89 (t,*J*=10.4 Hz, 2 H) 3.34 - 3.44 (m, 4 H) 3.70 (d, *J*= 12.7 Hz, 2 H) 4.79 (d, *J*=6.3 Hz, 2 H) 7.3 9 (td, *J*=8.3, 2.5 Hz, 1 H) 7.64 - 7.72 (m, 3 H) 8.44 (dd, *J*=8.4, 1.1 Hz, 1 H) 8.59 (t, *J*=5.6 Hz, 1 H) 9.00 (t, *J*=6.3 Hz, 1 H) 9.05 (dd, *J*=4.1, 1.1 Hz, 1 H) 12.72 (br. s., 1 H)

### Compound 9

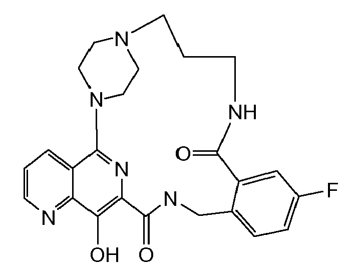

This compound was prepared in an analogous fashion as described for Examples 4.1 ― 4.5 and Compound 8.
1H NMR (300 MHz, DMSO-d₆) δ ppm 1.56 - 1.68 (m, 2 H) 2.43 (d, *J=*11.0 Hz, 2 H) 2.63 (br. s., 2 H) 2.79 (t, *J=*11*.*0 Hz, 2 H) 3.36 - 3.55 (m, 4 H) 3.71 (d, *J=*12.8 Hz, 2 H) 4.89 (d, *J*=3.0 Hz, 2 H) 7.37 (td, *J*=8.0, 2.5 Hz, 1 H) 7.57 - 7.65 (m, 2 H) 7.70 (dd, *J*=8.5, 4.1 Hz, 1 H) 8.42 (d, *J*=8.5 Hz, 1 H) 8.67 - 8.78 (m, 2 H) 9.05 (d, *J*=4.1 Hz, 1 H) 11.28 (br. s., 1 H)

### Compound 10

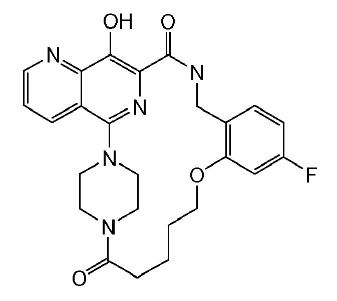

The protected macrocycle from Example 5.4 (0.2 gr) was dissolved in TFA (5 ml) and refluxed for 2 h. The solvent was removed under vacuum. The residue was triturated with saturated NaHCO₃ for 15 min., and the solid was collected by filtration. The precipitate was purified by prep. TLC (eluent: CH₂Cl₂/ methanol, 10:1). The residue was purified by prep. HPLC (C 18, eluent: MeOH/H₂O/TFA, 50:50:0.5) . The collected fractions were combined, concentrated under vacuum, basified to pH=7∼8 with saturated NaHCO₃ and extracted with CH₂Cl₂. The solvent was removed under vacuum, and the residue was recrystallized from CH₃CN to afford the title compound as a solid (6 mg).
1H NMR (400 MHz, CHLOROFORM-*d*) δ ppm 1.75 - 1.86 (m, 1 H) 2.02 - 2.24 (m, 3 H) 2.33 - 2.39 (m, 1 H) 2.77 - 2.89 (m, 2 H) 3.07 (dt, *J*=11.9, 2.8 Hz, 1 H) 3.13 - 3.25 (m, 2 H) 3.61 - 3.69 (m, 1 H) 3.76 - 3.84 (m, 1 H) 3.97 - 4.04 (m, 1 H) 4.10 - 4.16 (m, 1 H) 4.18 - 4.24 (m, 1 H) 4.53 (dd, *J*=14.2, 6.8 Hz, 1 H) 4.59 - 4.65 (m, 1 H) 4.67 (dd, *J*=14.2, 6.8 Hz, 1 H) 6.61 - 6.67 (m, 2 H) 7.32 (t, *J*=7.2 Hz, 1 H) 7.61 (dd, *J*=8.4, 4.2 Hz, 1 H) 8.60 (dd, *J*=8.4, 1.6 Hz, 1 H) 8.67 (t, *J*=6.8 Hz, 1 H) 9.13 (dd, *J*=4.2, 1.6 Hz, 1 H) 12.82 (s, 1 H)

### Compound 11

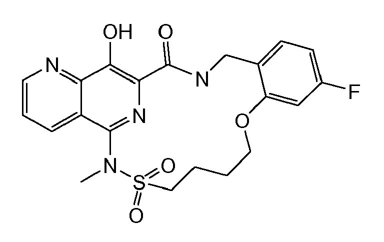

To a solution of the macrocyle obtained in Example 6.2 (0.42 mmol) and sodium iodide (0.92 mmol) in acetonitrile (2 ml) and toluene (2 ml) was added SiCl₄ (0.92 mmol)) at 0°C under nitrogen atmosphere. After completion of the reaction, water (10 ml) and methanol (10 ml) were added, followed by extraction with CH₂Cl₂ (3* 10 ml). The combined organic layers were washed with brine (15 ml) and dried over Na₂SO₄, filtered and evaporated under reduced pressure.
The residue was washed with ethyl ether and CH₃CN. The title compound was obtained by filtration (42 mg).
1H NMR (400 MHz, CHLOROFORM-*d*) δ ppm 2.04 (br. s., 2 H) 2.46 (br. s., 2 H) 3.38 - 3.45 (m, 5 H) 4.08 (br. s., 2 H) 4.61 (d, *J*=5.5 Hz, 2 H) 6.59 - 6.72 (m, 2 H) 7.34 (t, *J*=7.0 Hz, 1 H) 7.70 (dd, *J*=8.0, 4.3 Hz, 1 H) 8.63 (d, *J*=8.0 Hz, 2 H) 9.18 (br. s., 1 H) 13.33 (s, 1 H)

### Compound 12

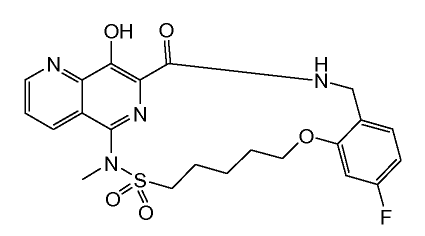

This compound was prepared in an analogous fashion as described in Compound 11, using Example 7.2 as the starting material.
1H NMR (400 MHz, CHLOROFORM-*d*) δ ppm 1.83 (quin, *J*=7.2 Hz, 2 H) 2.05 (quin, *J*=5.7 Hz, 2 H) 2.21 (br. s., 2 H) 3.31 (t, *J*=8.0 Hz, 2 H) 3.41 (s, 3 H) 4.19 (t, *J*=4.8 Hz, 2 H) 4.66 (d, *J*=6.0 Hz, 2 H) 6.63 - 6.73 (m, 2 H) 7.34 (t, *J*=7.1 Hz, 1 H) 7.69 (dd, *J*=8.3, 4.0 Hz, 1 H) 8.29 (t, *J*=6.0 Hz, 1 H) 8.63 (d, *J*=8.3 Hz, 1 H) 9.18 (d, *J*=4.0 Hz, 1 H) 13.58 (s, 1 H)

### B. BIOLOGICAL ACTIVITY OF COMPOUNDS OF FORMULA (I)

The anti-FIV activity of the compounds was tested in primary feline thymocytes isolated form specific pathogen free cats, and in Crandell feline kidney (CRFK) cells.

### Assay 1 - inhibitory activity on FIV replication wild type

### Feline thymocytes

Feline thymocytes were maintained in complete Iscove's Modified Medium (Iscoves medium supplemented with GlutamaxI (Invitrogen/Gibco BRL), 10% inactivated fetal calf serum, antibiotics (100 IU/ml penicillin and 100 µg/ml streptomycin) 2-Mercaptoethanol (50µM) and 100 IU/ml human recombinant IL-2 (Chiron). Cells were seeded into a 96 well plate (5x10⁴ cells per well) and pre-incubated with four-fold dilutions of the antiviral compound. Plates were incubated for 1 hour in a humidified incubator with a 5% CO₂ atmosphere at 37°C. Subsequently, cells were inoculated with 100 TCID50 of FIV strain Thymo-113 in the presence of the different concentrations of compound. After 2 hours cells were washed twice in Iscove's medium and new medium containing the different concentrations of compound were added. After incubation for 6 days the supernatant was harvested and the amount of virus within the supernatant determined using a FIV p24 ELISA. The 50% effective concentration (EC50) was defined as the concentration of the compound required to reduce the antigen production by 50% compared to untreated controls.

The EC50 for compound (6) was 20 nM.

### CRFK cells

Crandell Feline Kidney (CRFK) cells were maintained in DMEM (Dulbecco's Modified Eagle's Medium (Lonza) supplemented with, 10% inactivated fetal calf serum, antibiotics (100 IU/ml penicillin and 100 µg/ml streptomycin). Cells were seeded into a 96 well plate (5x10⁴ cells per well) and pre-incubated with four-fold dilutions of the antiviral compound for 1 hours. The CrFK cells were washed with PBS DEAE 50mg/l, inoculated with 100 TCID50 of FIV strain Utrecht-113 in the presence of the different concentrations of compound and subsequently incubated for 2 hours in a humidified incubator with a 5% CO₂ atmosphere at 37°C. After 2 hours cells were washed twice in DMEM medium and new medium containing the different concentrations of compound were added. After incubation for 6 days the supernatant was harvested and the amount of virus within the supernatant determined using a FIV p24 ELISA. The 50% effective concentration (EC50) was defined as the concentration of the compound required to reduce the antigen production by 50% compared to untreated controls.

The EC50 for compound (6) was 20 nM.

### Assay 2 - cellular cytotoxic activity

The cytotoxicity of the compounds was determined in parallel to the experiments under assay 1 on mock-infected feline thymocytes respectively CRFK cells cultured in the presence or absence of different concentrations of the compound.
After 6 days of incubation cell viability of the CRFK cells was measured by using a colorimetric MTT assay. A solution of 3-(4,5-dimethylthiazol-2-yl)-2,5-diphenyltetrazolium bromide (MTT) in PBS at a final concentration of 0,5 mg/ml was added to each well. After 2 hours incubation at 37 C and 5% CO2, the medium containing the MTT was removed and the cells were lysed by addition of 200 µl DMSO to each well. Following 10 minutes incubation at room temperature on a rocking plate, the optical density (OD) values at 570 nM were determined. The compound concentration that decreased the viability of 50% of the cells was defined as the 50% cytotoxic concentration (CC50).

The CC50 for compound (6) was > 10 nM.

Cytotoxicity on feline thymocytes was determined by trypan blue exclusion staining whereby the CC50 for compound (6) was measured as > 10 nM.

## Claims

1. A compound of formula (I), including the stereochemically isomeric forms thereof, and pharmaceutically acceptable salts thereof, 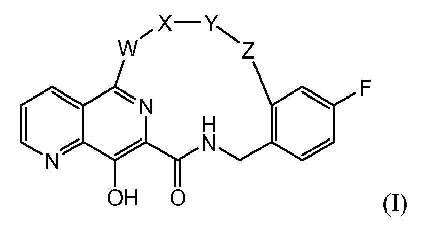
wherein W is -NH-, -N(CH₃)- or piperazine,
X is a bond, -C(=O)- or -S(=O)₂-,
Y is C₃₋₇alkylene, and
Z is -NH-C(=O)- or -O-;
for use in the treatment and prevention of feline immunodeficiency virus.

2. The compound according to claim 1 wherein W is -NH- or -N(CH₃)-.

3. The compound according to claim 1 or 2 wherein X is a bond or -S(=O)₂-.

4. The compound according to any of claims 1 to 3 wherein Y is C₄₋₅alkylene.

5. The compound according to claim 1 wherein X is -C(=O)- and Y is C₃₋₄alkyl when W is piperazine.

6. The compound according to any of claims 1 to 5 wherein Z is ―O-.

7. The compound according to any of claims 1 to 5 wherein Z is -NH-C(=O)-, in particular wherein the nitrogen of -NH-C(=O)- is connected to Y.

8. The compound according to any of claims 1 to 7 wherein the -W-X-Y-Z- linker is 8 or 9 atoms long.

9. The compound according to claim 1 wherein -W-X-Y-Z- is selected from
---NH-C₅-₇akylene-NH-C(=O)---,
---N(CH₃)-S(=O)₂-C₄-sakylene-NH-C(=O)---,
--- N(CH₃)-S(=O)₂-C₄₋₅alkylene-O---, 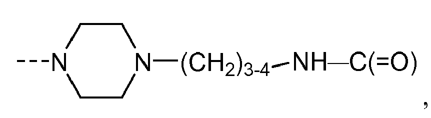
and,

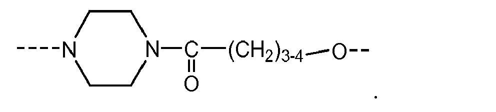

10. The compound as claimed in claim 1 having the formula

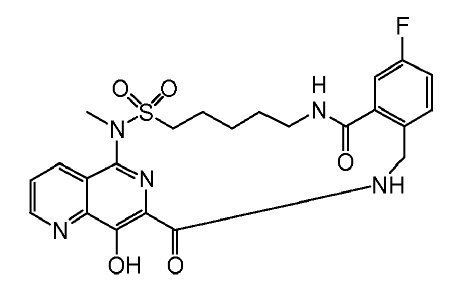

11. A product comprising a compound of formula (I) as defined in any one of claims 1 to 10 and an anti-viral agent as a combined preparation for simultaneous, separate or sequential use in the treatment or prophylaxis of feline immunodeficiency virus infection.

12. A method of treating or preventing infection of feline immunodeficiency virus in a feline animal wherein said method comprises the administration to said feline animal of a therapeutically effective amount of a compound of formula (I) according to any of claims 1 to 10.
